# EUROPEAN PATENT APPLICATION

(11) **EP 2 784 158 A1**
(43) Date of publication of application: **01.10.2014**
(21) Application number: 12851503.8
(22) Date of filing: 22.11.2012
(51) Int. Cl.: C12N 15/09, A01H 1/00, A01H 5/00

(54) **PLANT TRANSFORMENT, TRANSFORMATION METHOD FOR PLANT, AND VECTOR USED IN SAID METHOD**

(30) Priority: 25.11.2011 JP 2011258107
(71) Applicant: National Institute of Agrobiological Sciences, Tsukuba-shi, Ibaraki 305-8602 (JP)
(72) Inventor: TABEI, Yutaka, Tsukuba-shi Ibaraki 305-8602 (JP); OKUZAKI, Ayako, Tsukuba-shi Ibaraki 305-8602 (JP)
(74) Representative: Cohausz & Florack
(86) International application number: PCT/JP2012/080355
(87) International publication number: WO 2013/077420

(57) **Abstract**

The present invention provides a method for transforming organelles having an own genomic DNA, the method enabling efficient production of highly homoplasmic plant cells, in which most of the organelles are transformed, and so forth. The method comprises the steps of: expressing in plant cells a fusion protein containing a function inhibiting factor of the organelles and a transit signal peptide to the organelles; introducing into the genomic DNA of the organelles of the plant cells an expression cassette comprising DNAs encoding a restoring factor of the organelles and a factor desired to be expressed in the organelles; and allowing the function inhibiting factor to destroy the organelles, in which the expression cassette is not introduced, in the plant cells.

SEQUENCE LISTING

<110>  NATIONAL INSTITUTE OF AGROBIOLOGICAL SCIENCES
<120>  TRANSFORMANT OF PLANT, METHOD OF TRANSFORMING PLANT, AND VECTOR USING
THE METHOD
<130>  IBPF12-541WO
<150>  JP2011/258107
<151>  2011-11-25
<160>  18
<170>  PatentIn version 3.1

<210>  1
<211>  28
<212>  DNA
<213>  Artificial
<220>
<223>  Artificially synthesized primer sequence
<400>  1
gtgtcgacgc acaggttatc aacacgtt                                          28

<210>  2
<211>  32
<212>  DNA
<213>  Artificial
<220>
<223>  Artificially synthesized primer sequence
<400>  2
gtttaattaa ttatctgatt tttgtaaagg tc                                     32

<210>  3
<211>  28
<212>  DNA
<213>  Artificial
<220>
<223>  Artificially synthesized primer sequence
<400>  3
agctcgagat ggcttcctct atgctctc                                          28

<210>  4
<211>  28
<212>  DNA
<213>  Artificial
<220>
<223>  Artificially synthesized primer sequence
<400>  4
ttgtcgacgc agttaactct tccgccgt                                          28

<210>  5
<211>  21
<212>  DNA
<213>  Artificial
<220>
<223>  Artificially synthesized primer sequence
<400>  5
gagcctgacc tattgcatct c                                                 21

<210>  6
<211>  20
<212>  DNA
<213>  Artificial
<220>
<223>  Artificially synthesized primer sequence
<400>  6

gtacttctac acagccatcg                                                    20

<210>  7
<211>  31
<212>  DNA
<213>  Artificial
<220>
<223>  Artificially synthesized primer sequence
<400>  7
gtcggatcca tgaaaaaagc agtcattaac g                                       31

<210>  8
<211>  31
<212>  DNA
<213>  Artificial
<220>
<223>  Artificially synthesized primer sequence
<400>  8
cccggatcct taagaaagta tgatggtgat g                                       31

<210>  9
<211>  28
<212>  DNA
<213>  Artificial
<220>
<223>  Artificially synthesized primer sequence
<400>  9
gtccgcggcg gaaagaacac caacggcg                                           28

<210>  10
<211>  29
<212>  DNA
<213>  Artificial
<220>
<223>  Artificially synthesized primer sequence
<400>  10
tcgcggccgc agctgggcca tcctggact                                          29

<210>  11
<211>  27
<212>  DNA
<213>  Artificial
<220>
<223>  Artificially synthesized primer sequence
<400>  11
gagtcgacag ctgcgccagg gaaaaga                                            27

<210>  12
<211>  27
<212>  DNA
<213>  Artificial
<220>
<223>  Artificially synthesized primer sequence
<400>  12
ccgggcccaa gccactgcct atgagtc                                            27

<210>  13
<211>  42
<212>  DNA
<213>  Artificial
<220>
<223>  Artificially synthesized oligonucleotide sequence
<400>  13

tcgacatcga tacgtacgtt aattaacctg caggcccggg cc 42

<210> 14
<211> 63
<212> DNA
<213> Phage T7
<400> 14
gggagaccac aacggtttcc ctctagaaat aattttgttt aactttaaga aggagatata 60

cat 63

<210> 15
<211> 30
<212> DNA
<213> Artificial
<220>
<223> Artificially synthesized primer sequence
<400> 15
atgcggccgc ttctagttgg atttgctccc 30

<210> 16
<211> 25
<212> DNA
<213> Artificial
<220>
<223> Artificially synthesized primer sequence
<400> 16
agtacgtaga caaagcggat tcgga 25

<210> 17
<211> 26
<212> DNA
<213> Artificial
<220>
<223> Artificially synthesized primer sequence
<400> 17
acccgggaga ccacaacggt ttccct 26

<210> 18
<211> 31
<212> DNA
<213> Artificial
<220>
<223> Artificially synthesized primer sequence
<400> 18
aggatcccat atgtatatct ccttcttaaa g 31

## Description

### [Technical Field]

The present invention relates to a method for transforming organelles having an own genomic DNA in a plant. More specifically, the present invention relates to a method for transforming the organelles, the method comprising; expressing in plant cells a fusion protein containing a function inhibiting factor of the organelles and a transit signal peptide to the organelles; introducing into the genomic DNA of the organelles of the plant cells an expression cassette comprising a DNA encoding a restoring factor of the organelles and a DNA encoding a factor desired to be expressed in the organelles; and allowing the function inhibiting factor to destroy the organelles, in which the expression cassette is not introduced, in the plant cells.

### [Background Art]

Organelles such as plastids and mitochondria have own genomic DNAs, and transformants thereof can be produced by genetic modification as in the case of the nuclear genomic DNA. In the genetic modification of organelles such as plastids and mitochondria, since an introduced gene is maternally inherited, there is a low risk of releasing the transformants into the environment by pollens. Moreover, unlike a gene introduced in the nuclear genome, gene silencing does not occur in organelles such as plastids and mitochondria. Hence, stable expression of the introduced gene is anticipated. Further, since having potent protein synthesis ability and protein storage ability, plastids are suitable for mass production of proteins. For these reasons, the transformation method is expected to greatly contribute to production and development of biomass, functional food materials, pharmaceutical materials, and so forth. The first report regarding the transformation method by which a gene is introduced into the genomic DNA of chloroplasts, one type of plastids, was made on tobacco (NPL 1). Since then, researches have been in progress on various plants.

However, as to dicots such as tobacco (NPLs 2 to 4), potato (NPL 5), and eggplant (NPL 6), there are reports that homoplasmic plants having only transformed chloroplasts are obtained, but it is necessary to repeat tissue culturing from leaf segments several times. In addition, although rapeseed is a dicot, no homoplasmic plants have been obtained therefrom (NPL 7). As to monocots, although chloroplast transformants have been obtained only from rice, the resultants were all heteroplasmic individuals. It is considered to be difficult to eliminate the heteroplasmy even by long-term selection of cultured cells (NPLs 8 and 9) . As to monocots other than rice also, it is almost impossible to regenerate individuals from leaf segments of corn and other monocots, suggesting that it is difficult to select homoplasmic individuals.

Since as many as 100 or more of plastids and the like may be present in a single cell, when organelles having an own genomic DNA are transformed, heteroplasmic cells containing both transformed and wildtype organelles are produced at first in many cases as described above. Moreover, in some cases, chimeric plant tissues or plants are obtained, in which homoplasmic cells, heteroplasmic cells, and cells containing only wildtype plastids and the like are all present. Homoplasmic plants have an advantage that an introduced gene is expressed at high level, and are also characterized in that the target gene is inherited to all the seeds in the next generation by maternal inheritance. Meanwhile, in heteroplasmic or chimeric transformants, the level of expression of an introduced target gene in one plant individual is lower than that in one homoplasmic individual, so that there is a problem that the inheritance to the next generation is not uniform (NPLs 7 to 9) . So far, methods for transforming organelles having an own genomic DNA have been developed for many plants. Along with this, the development of a highly versatile method for eliminating the heteroplasmy and chimerism is also strongly desired. However, such an elimination method is yet to be developed.

On the other hand, a protein called barnase is known as an RNase derived from *Bacillus amyloliquefaciens,* one species of the genus *Bacillus.* Additionally, a protein called barstar is also known as an intracellular inhibiting factor capable of specifically binding to the barnase. Further, there are also reports that male sterile plants are produced by expressing barnase in male organs in plants (NPL 10), and that the restoration of the male sterility is possible using barstar (NPLs 11 to 13) . Nevertheless, there is no case in which barnase and barstar are utilized to function in organelles such as plastids.

### [Citation List]

### [Non Patent Literatures]

[NPL 1] Svab Z et al . , Proc Natl Acad Sci USA, 1990, vol. 87, pp. 8526 to 8530
[NPL 2] Svab Z et al . , Proc Natl Acad Sci USA, 1993, vol. 90, pp. 913 to 917
[NPL 3] Ruf S et al., Nature biotechnol, 2001, vol. 19, pp. 870 to 875
[NPL 4] Verma D et al., Plant Physiology, 2007, vol. 145, pp. 1129 to 1143
[NPL 5] Nguyen TT et al. , Plant Science, 2005, vol. 168, pp. 1495 to 1500
[NPL 6] Singh AK et al., Transgenic Res, 2010, vol. 19, pp. 113 to 119
[NPL 7] Chen L et al., Plant Cell Rep, 2010, vol. 29, pp. 371 to 381
[NPL 8] Khan MS et al., Nat Biotechnol, 1999, vol. 17, pp. 910 to 915
[NPL 9] Lee SM et al. , Mol Cells, 2006, vol. 21, pp. 401 to 410
[NPL 10] Mariani C et al., Nature, 1990, vol. 347, pp. 737 to 741
[NPL 11] Mariani C et al., Nature, 1992, vol. 357, pp. 384 to 387
[NPL 12] Burgess DG et al. , The Plant Journal, 2002, vol. 31, pp. 113 to 125
[NPL 13] Jagannath A et al. , Current Science, 2002, vol. 82, iss. 1, pp. 46 to 52

### [Summary of Invention]

### [Technical Problem]

The present invention has been made in consideration of the above-described problems in the conventional techniques. An object of the present invention is to provide a method for transforming organelles having an own genomic DNA in a plant, the method enabling efficient production of highly homoplasmic plant cells, in which most of plastids and the like are transformed, highly homoplasmic plants composed mostly of the plant cells, and so forth.

### [Solution to Problem]

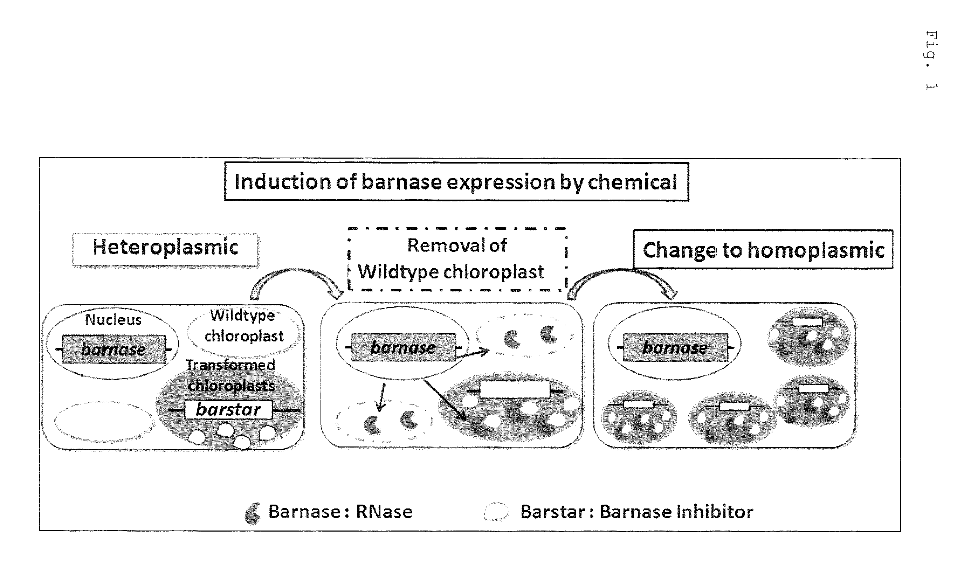

The present inventors have earnestly studied in order to achieve the above object. As a result, an RNase barnase gene, to which a transit signal peptide to chloroplasts is added, is introduced in the nuclear genome of a plant leaf, while a barstar gene for inhibiting barnase is introduced in the chloroplast genome, as shown in Fig. 1. Note that the barnase gene is expressed by an estradiol-inducible promoter. As a result, the present inventors have found out that even when the barnase expression is induced, the transformedchloroplasts having the barstar gene are not influenced by the enzyme, and only wildtype chloroplasts are destroyed. Moreover, the inventors have found out that such a method efficiently increases a proportion of the transformed chloroplasts in a plant cell, plant tissue, or plant, consequently efficiently producing homoplasmic plant cells, in which most of chloroplasts are transformed, and highly homoplasmic plants composed mostly of the plant cells, and other effects. These discoveries have led to the completion of the present invention.

Note that barnase has been utilized in plants mainly by expressing barnase specifically in a male organ of a plant, thereby disrupting pollens so as to induce sterility. Accordingly, the present invention has revealed for the first time effects of localizing barnase in organelles such as chloroplasts having an own genomic DNA.

Thus, the present invention relates to a method for transforming organelles having an own genomic DNA in a plant, a vector used in the method, a transformant produced by the method. More specifically, the present invention provides the following inventions.
(1) A method for transforming organelles having an own genomic DNA in a plant, the method comprising the steps of:
   expressing in plant cells a fusion protein containing a function inhibiting factor of the organelles and a transit signal peptide to the organelles;
   introducing into the genomic DNA of the organelles of the plant cells an expression cassette comprising a DNA encoding a restoring factor of the organelles and a DNA encoding a factor desired to be expressed in the organelles; and
   allowing the function inhibiting factor to destroy the organelles, in which the expression cassette is not introduced, in the plant cells.
(2) The method according to (1), wherein the function inhibiting factor is barnase, and the restoring factor is barstar.
(3) The method according to any one of (1) and (2), wherein the organelles are plastids.
(4) The method according to any one of (1) to (3) , wherein the expression of the function inhibiting factor is induced in response to a stimulus.
(5) At least one vector selected from the following (a) and (b) used in the method according to any one of (1) to (4) :
   (a) a vector comprising
      a promoter capable of functioning in the plant cells, and
      a DNA operably linked to the promoter and encoding the fusion protein; and
   (b) a vector comprising
      an expression cassette comprising a first promoter capable of functioning in the organelles, the DNA operably linked to the first promoter and encoding the restoring factor, a second promoter capable of functioning in the organelles, and the DNA operably linked to the second promoter and encoding the factor desired to be expressed in the organelles, and
      DNA sequences located on both sides of the expression cassette and having a homology with the genomic DNA specific to the organelles.
(6) A transformant produced by the method according to any one of (1) to (4).
(7) A transformant comprising at least one DNA selected from the following (a) and (b):
   (a) a promoter capable of functioning in the plant cells and a DNA operably linked to the promoter and encoding the fusion protein; and
   (b) a first promoter capable of functioning in organelles having an own genomic DNA in a plant, a DNA operably linked to the first promoter and encoding the restoring factor, a second promoter capable of functioning in the organelles, and a DNA operably linked to the second promoter and encoding the factor desired to be expressed in the organelles.
(8) A transformant that is any one of a progeny and a clone of the transformant according to any one of (6) and (7) .
(9) The transformant according to any one of (6) to (8), wherein the transformant is any one selected from the group consisting of a plant cell, a plant tissue, a plant organ, a plant, and a seed.
(10) A processed product of the transformant according to any one of (6) to (9) , the processed product comprising an expression product of the factor desired to be expressed in the organelles.

### [Advantageous Effects of Invention]

The present invention enables efficient production, in transforming organelles having own genomic DNAs in a plant, of homoplasmic plant cells, in which most of the organelles are transformed, highly homoplasmic plants composed mostly of the plant cells, and so forth. Particularly, in transforming plastids, a proportion of the transformed plastids in a plant cell, plant tissue, or plant is efficiently increased, thereby increasing a total level of expression of introduced exogenous genes per cell of an equivalent volume. This makes it possible to produce plastid-transformed plants having a high substance-producing ability.

### [Brief Description of Drawings]

[Fig. 1] Fig. 1 is a schematic drawing for illustrating one embodiment of a transformation method of the present invention.

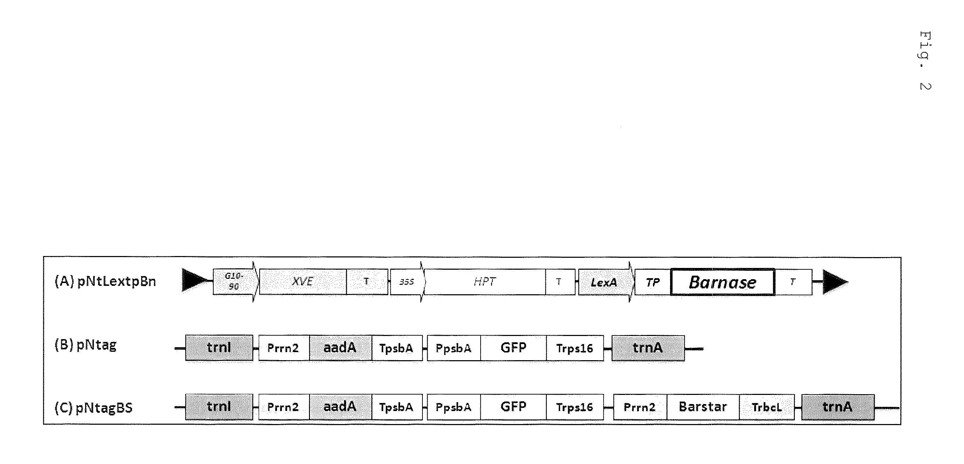

[Fig. 2] Fig. 2 is a schematic drawing showing configurations of genes used in the transformation method of the present invention. (A) shows a portion of the configuration of a vector (pNtLextpBn) for introducing into the nucleus a DNA encoding barnase, to which a transit signal to chloroplasts is added. "G10-90" indicates a synthetic promoter (see Ishige F, Plant J, 1999, vol. 18, iss. 4, pp. 443 to 448) . "XVE" indicates a DNA encoding an estrogen receptor fused with a transcription activating factor. "35S" indicates a 35S promoter. "HPT" indicates a hygromycin-resistance gene. "LexA" indicates an estradiol-inducible promoter. "TP" indicates a DNA encoding a chloroplast transit peptide of *Arabidopsis thaliana-derived RbcS.* "Barnase" indicates a DNA encoding barnase. "T" indicates a terminator. (C) shows a portion of the configuration of a vector (pNtagBS) for expressing barstar in chloroplasts. "trnI" and "trnA" respectively indicate flanking regions (a region specified under ACCESSION No. Z00044: 103417-105335 (1.9 kb), and a region specified under ACCESSION No. Z00044: 105330-106944 (1.6 kb) ) of a trnI gene and a trnA gene of tobacco chloroplasts. "Prrn2" indicates a tobacco chloroplast-derived rRNA operon promoter, to which a phage-derived T7G10 sequence is added. "psbA" indicates a tobacco chloroplast-derived photosynthesis gene psbA promoter. Moreover, "aadA" indicates a spectinomycin-resistance gene. "Barstar" indicates a DNA encoding barstar. "GFP" indicates a DNA encodingGFP. "TpsbA","Trps16", and "TrbcL"respectively indicate terminators of a psbA gene, a DNA encoding tobacco chloroplast-derived ribosomal protein S16, and a tobacco chloroplast-derived rps16 gene. (B) shows a portion of the configuration of a vector (pNtag; a control vector of the pNtagBS) for expressing GFP in chloroplasts.

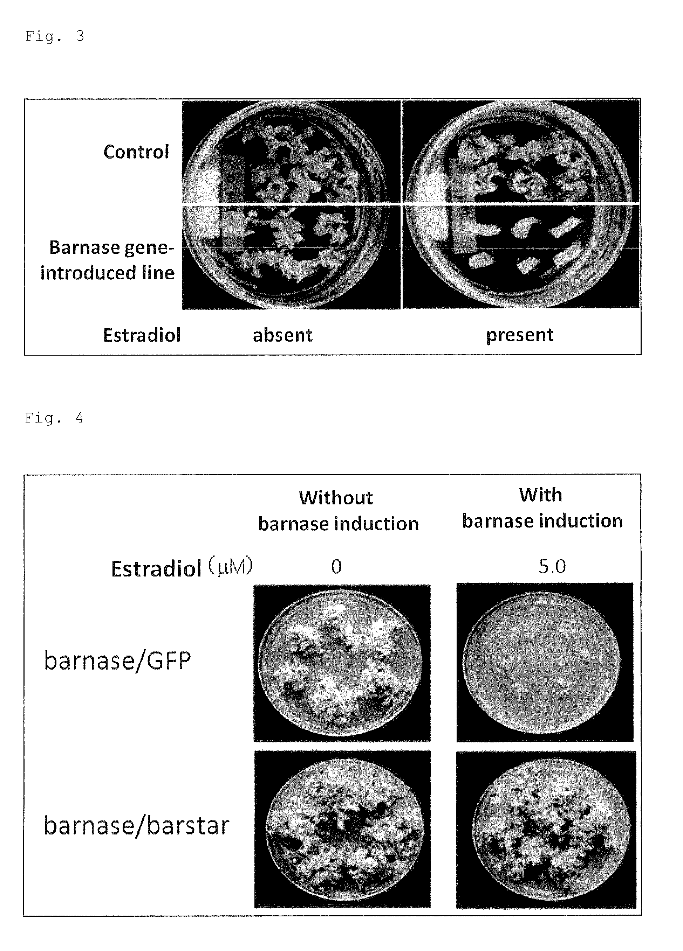

[Fig. 3] Fig. 3 shows photographs for illustrating that barnase expression was induced in pNtLextpBn-introduced lines by performing an estradiol treatment, inhibiting leaf segments of the lines from forming adventitious shoots.

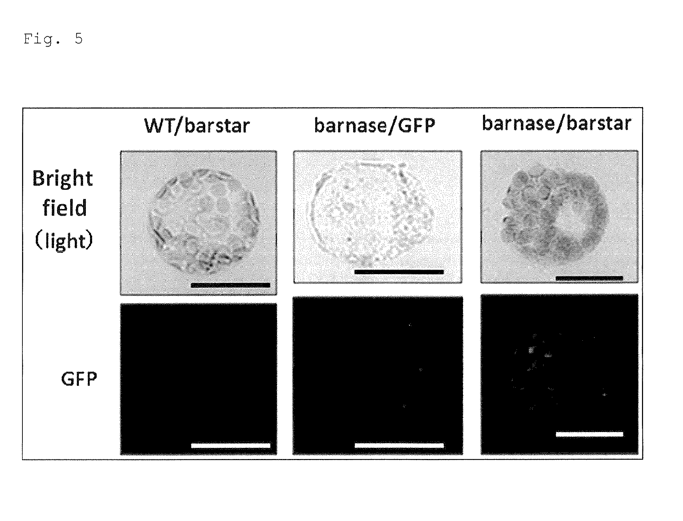

[Fig. 4] Fig. 4 shows photographs for illustrating that barstar introduced into chloroplasts inhibited barnase from suppressing re-differentiated adventitious shoot formation. "barnase/GFP" shows the result of tobacco having the barnase gene introduced in the nucleus and the pNtag introduced in the chloroplast. "barnase/barstar" shows the result of tobacco having barnase introduced in the nucleus and the pNtagBs introduced in the chloroplast (the same applies to Figs. 5 to 8).

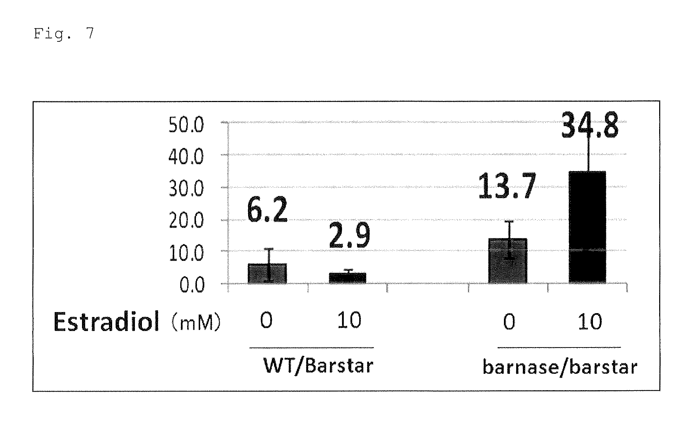

[Fig. 5] Fig. 5 shows micrographs of protoplast cells, showing the result of observing a state in which chloroplasts were disrupted by barnase and a state in which the disruption was suppressed by barstar. "WT/barstar" shows the result of tobacco having the wildtype nucleus and the pNtagBs introduced in the chloroplast (the same applies to Fig. 7). Additionally, the scale bar represents 50 µm.

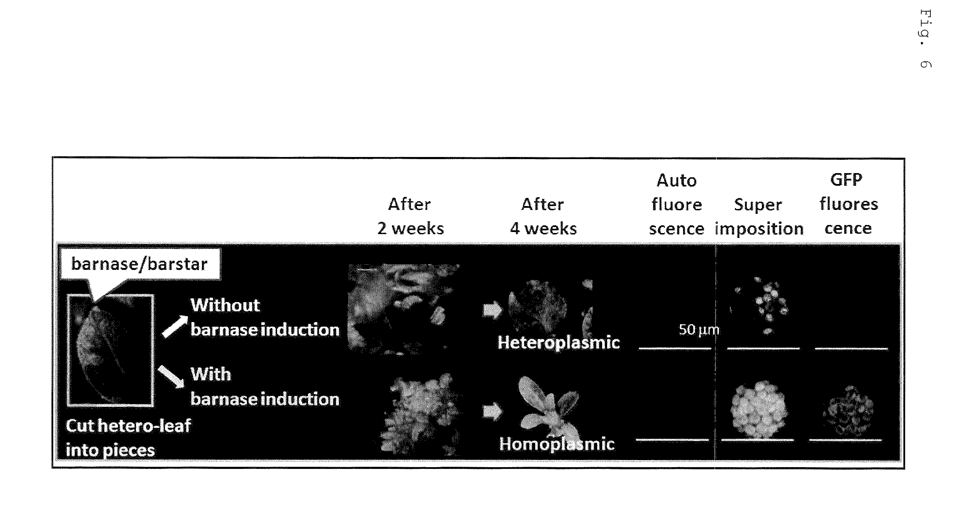

[Fig. 6] Fig. 6 shows a schematic representation for illustrating a step of examining the influence on the homoplasmy of re-differentiated adventitious shoots by the presence or absence of barnase induction, fluorescence microphotographsofre-differentiatedadventitiousshoots obtained at 2 weeks and 4 weeks after culturing was started in the step, and fluorescence microphotographs of protoplast cells obtained at the 4 weeks.
[Fig. 7] Fig. 7 is a graph for illustrating proportions of homoplasmic adventitious shoots produced.

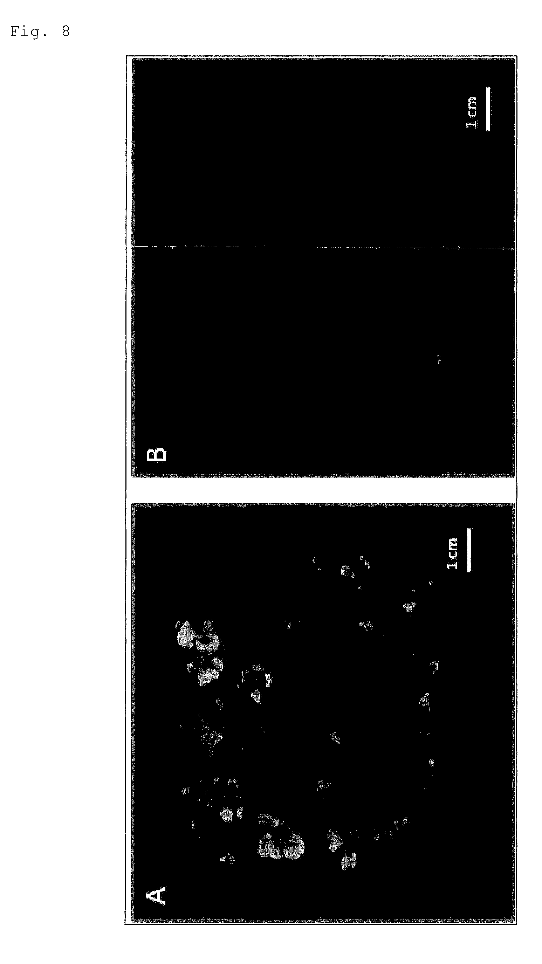

[Fig. 8] Fig. 8 shows micrographs for observing GFP fluorescence in secondary re-differentiated adventitious shoots prepared from heteroplasmic primary re-differentiated individuals. Note that, in the figure, the panel A shows the result of observing secondary re-differentiated adventitious shoots prepared from primary re-differentiated individuals Bn/Bs-6 of the barnase/barstar, and the panel B shows the result of observingsecondaryre-differentiatedadventitiousshoots prepared from primary re-differentiated individuals WT/Bs-5 of the WT/barstar.

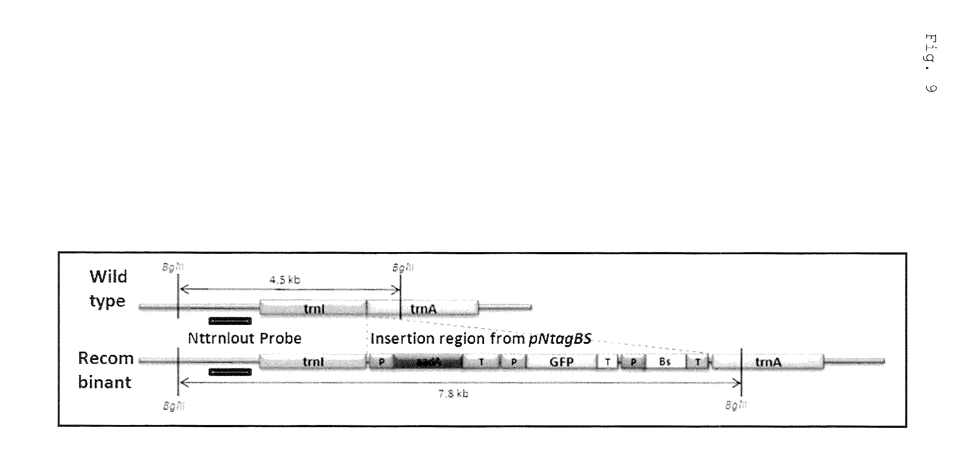

[Fig. 9] Fig. 9 is a schematic drawing of genomic regions of the trnI and trnA genes of tobacco chloroplasts. Note that, in Fig. 9, the upper portion (wildtype) shows a genomic region of wildtype tobacco chloroplasts, and the lower portion (recombinant) shows a genomic region of the tobacco chloroplasttransformedbythepNtagBS. Moreover, "BglII" indicates a cleavage site of a restriction enzyme BglII in each genomic region. "NttrnIout probe" indicates a site where an endogenous chloroplast DNA-derived sequence NttrnIout probe hybridizes.

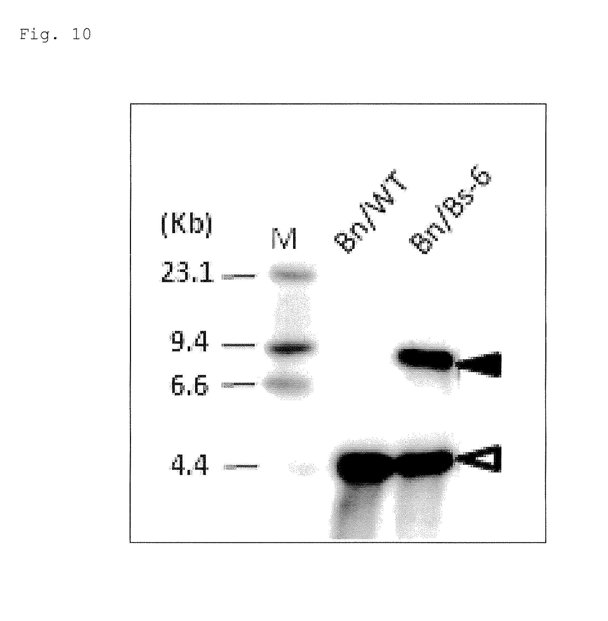

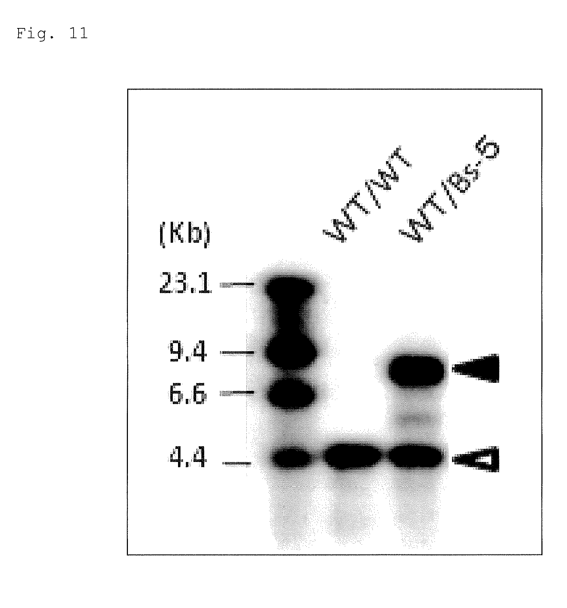

[Fig. 10] Fig. 10 is a photograph showing the result of analyzing a chloroplast transformation material barnase/WT (Bn/WT) and the heteroplasmic primary re-differentiated individuals (Bn/Bs-6) by Southern blotting using the NttrnIout probe. Note that, in the figure, "M" indicates a size marker, the black triangle indicates a band (7.8-kb signal) derived from the tobacco chloroplast genome transformed by the pNtagBS, and the white triangle indicates a band (4.5-kb signal) derived from the wildtype tobacco chloroplast genome (the same applies to Figs. 11 to 13).
[Fig. 11] Fig. 11 is a photograph showing the result of analyzing a chloroplast transformation material wildtype individuals (WT/WT) and the heteroplasmic primary re-differentiated individuals (WT/Bs-5) by Southern blotting using the NttrnIout probe.

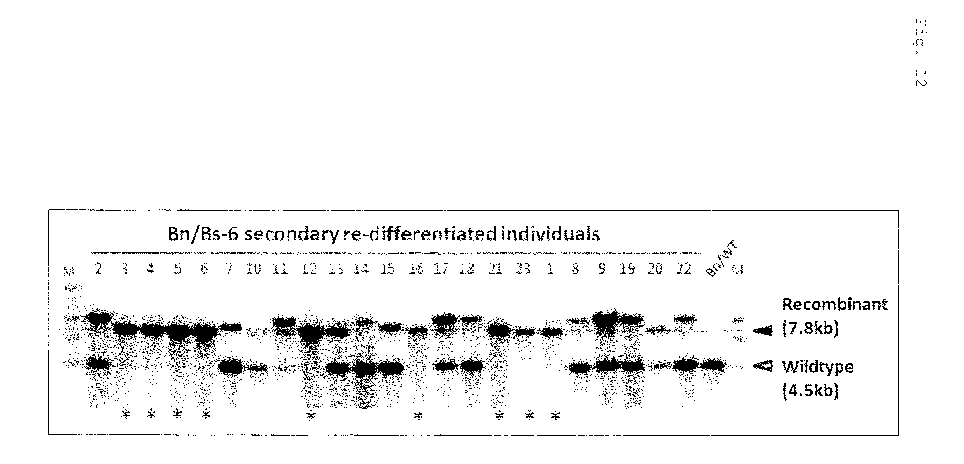

[Fig. 12] Fig. 12 is a photograph showing the result of analyzing secondary re-differentiated individuals prepared from the heteroplasmic primary re-differentiated individuals Bn/Bs-6 by Southern blotting using the NttrnIout probe. Numbers 1 to 22 denoted in lanes indicate the numbers (individual identification numbers) assigned to the individuals. The asterisk indicates a plant composed of homoplasmic cells in quite a high proportion, in which the band derived from the wildtype tobacco chloroplast genome is hardly detected.

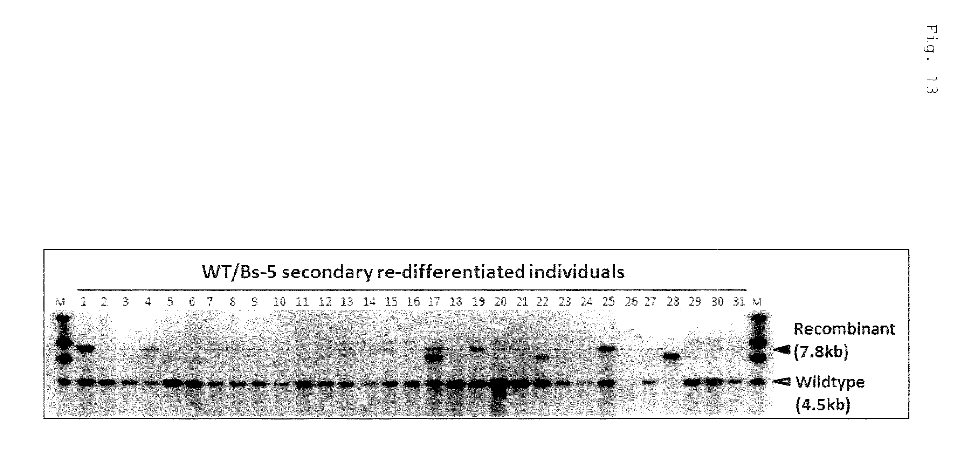

[Fig. 13] Fig. 13 is a photograph showing the result of analyzing secondary re-differentiated individuals prepared from the heteroplasmic primary re-differentiated individuals WT/Bs-5 by Southern blotting using the NttrnIout probe. Numbers 1 to 31 denoted in lanes indicate individual identification numbers.

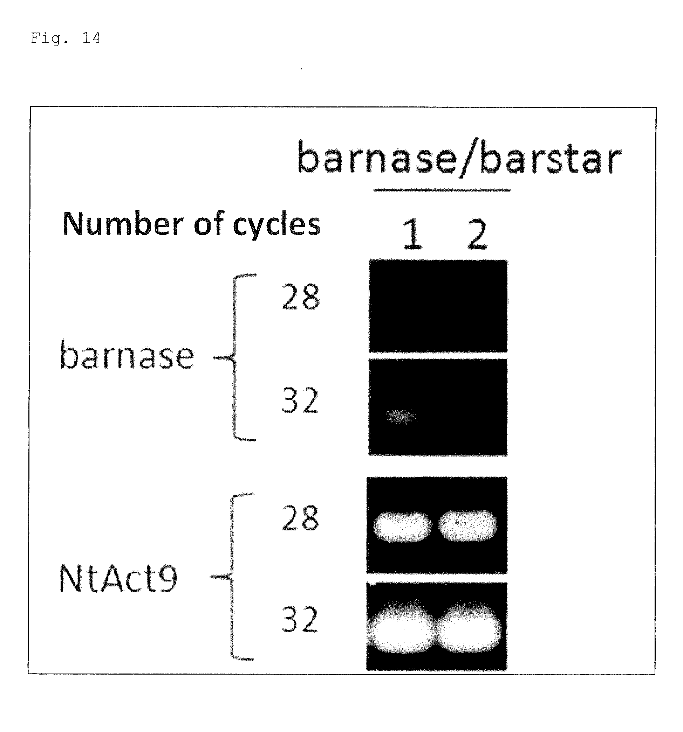

[Fig. 14] Fig. 14 shows electrophoresis images, showing the result of examining the expression of the barnase gene not induced by estradiol in the "barnase/barstar" by a RT-PCR analysis. "NtAct9" shows the result of an actin gene amplified by RT-PCR as an internal standard. "1" and "2" show the results of different "barnase/barstar" individuals not subjected to the estradiol treatment.

### [Description of Embodiments]

### <Transformation Method>

A transformation method of the present invention is a method for transforming organelles having an own genomic DNA in a plant, the method comprising the steps of:
expressing in plant cells a fusion protein containing a function inhibiting factor of the organelles and a transit signal peptide to the organelles;
introducing into the genomic DNA of the organelles of the plant cells an expression cassette comprising a DNA encoding a restoring factor of the organelles and a DNA encoding a factor desired to be expressed in the organelles; and
allowing the function inhibiting factor to destroy the organelles, in which the expression cassette is not introduced, in the plant cells.

Additionally, as described in Examples later, the method enables efficient production of highly homoplasmic plant cells, in which most of the organelles are transformed, highly homoplasmic plants composed mostly of the plant cells, and so forth.

Note that, in the present invention, the term "homoplasmic" refers to a state in which a single genotype of the DNA of the organelles is present in a cell, tissue, organ, individual, or the like. Unless otherwise specifically stated, "homoplasmic" refers to a state in which only the organelles having a transformed genomic DNA are present in a cell, tissue, organ, individual, or the like. Specifically, unless otherwise specifically stated, a "homoplasmic plant cell" refers to a plant cell having only the organelles having only a transformed genomic DNA, and homoplasmic plant tissue, plant organ, or plant refers to a plant tissue, plant organ, or plant composed only of homoplasmic cells. Moreover, a "highly homoplasmic plant cell" refers to a plant cell having the organelles having a transformed genomic DNA in a high proportion (generally 80% or more, preferably 90% or more, more preferably 95% or more, particularly preferably 99% or more), and "highly homoplasmic plant tissue, plant organ, or plant" refers to a plant tissue, plant organ, or plant, which are composed of homoplasmic cells and/or highly homoplasmic plant cells in a high proportion (generally 80% or more, preferably 90% or more, more preferably 95% or more, particularly preferably 99% or more).

In the present invention, the term "heteroplasmic" refers to a state in which different genotypes of the DNA of the organelles are present in a cell, tissue, organ, individual, or the like. Specifically, a "heteroplasmic plant cell" refers to a plant cell, in which both organelles having a transformed genomic DNA and organelles having a wildtype genomic DNA are present, and "heteroplasmic plant tissue or plant" refers to a plant tissue, organ, or plant, which are composed of heteroplasmic plant cells, or a plant tissue, plant organ, or plant, in which both homoplasmic plant cells (plant cells having only the organelles having a transformed genomic DNA, or cells having only the organelles having a wildtype genomic DNA) and heteroplasmic cells are present. Moreover, in the present invention, a "state in which both homoplasmic plant cells (plant cells having only the organelles having a transformed genomic DNA, or cells having only the organelles having a wildtype genomic DNA) and heteroplasmic cells are present" may also be referred to as "chimeric".

In the present invention, the "organelle having an own genomic DNA in a plant" means an organelle, that is, a plastid or a mitochondrion, having an own genomic DNA in a plant cell, other than the nucleus . Note that a plastid exists as an organ called a chloroplast in a green tissue, or exists as an organ such as an amyloplast, an etioplast, an elaioplast, a chromoplast, or a leucoplast in the other plant tissues. Moreover, all the plastids in a plant are obtained from undifferentiated plastids called proplastids after the differentiation. Further, these plastids mutually differentiate or re-differentiate, and change the morphology depending on the function necessary in each plant tissue. Nevertheless, the plastid DNA does not change in accordance with these morphological changes; in addition, even in a case where exogenous genes are to be inserted into the plastid DNA, the insertion of such exogenous genes are not changed in accordance with these morphological changes.

In the present invention, a "plant" is not particularly limited, and examples thereof include dicots such as tobacco, potato, eggplant, and rapeseed, and monocots such as rice, wheat, barley, and corn.

Moreover, in the present invention, the "factor desired to be expressed in the organelles" is not particularly limited. Further, the expression cassette may comprise only one DNA or multiple DNAs for encoding such a factor.

The "function inhibiting factor of the organelles" according to the present invention is a factor, which inhibits the function of the organelles, thereby suppressing the formation, development, maintenance, and growth of the organelles, and consequently destroying the organelles. Such a function inhibiting factor is preferably a factor involved in essential functions of the organelles, such as replication, transcription, and segregation of DNA, and synthesis and metabolism of protein, or a factor targeting the functions. Examples of the factor include RNases such as barnase and RNase T1, DNases, proteases such as papain, adenine nucleotide translocator, and DTA (diphtheria toxin A-chain).

Among these, the "function inhibiting factor of the organelles" according to the present invention is preferably barnase, from the viewpoint of usefulness in combination with barstar capable of specifically binding to the corresponding function inhibiting factor to thereby efficiently suppress the function of the factor as described in Examples later.

Note that the "barnase" according to the present invention is an RNase derived from *Bacillus amyloliquefaciens,* and is typically a protein (a protein having an amino acid sequence from positions 40 to 149 in the amino acid sequence specified under GenBank ACCESSION No: AAA86441.1; hereinafter, may also be referred to as "wildtype barnase"), which is obtained by excluding a secretory transit signal region (amino acid residues from positions 1 to 26 in the amino acid sequence specified under GenBank ACCESSION No: AAA86441.1) from a protein (a protein having the amino acid sequence specified under GenBank ACCESSION No: AAA86441.1) encoded by a DNA sequence specified under GenBank ACCESSION No: M14442.

Moreover, the amino acid sequence of the "barnase" according to the present invention may be mutated naturally (i.e., non-artificially). Meanwhile, a mutation can also be introduced artificially. Thus, the present invention comprises such mutants also, as long as the RNase activity and the binding ability to barstar are substantially equivalent to those of the wildtype barnase.

The barnase mutants include proteins having an amino acid sequence from positions 40 to 149 in the amino acid sequence specifie dunder GenBank ACCESSION No:AAA86441.1, in which one or more amino acids are substituted, deleted, added, and/or inserted. Herein, the term "more" refers to generally 20 amino acids or less, preferably 10 amino acids or less, more preferably several amino acids or less (for example, 5 amino acids or less, 3 amino acids or less, 2 amino acids or less, 1 amino acid).

Further, there are reports that, in barnase, the glutamic acid residue at position 99 in the amino acid sequence specified under GenBank ACCESSION No: AAA86441.1 is important for binding to the substrate RNA (see Katrin BASTYNS et al. , Biochem J. , 1994, vol. 300, pp. 737 to 742), and also that the glutamic acid residue at position 112 in the amino acid sequence is important for the binding to barstar (see Gideon Schreiber et al., J. Mol. Biol., 1997, vol. 270, pp. 111 to 122).

Note that the glutamic acid residue at position 99 and the glutamic acid residue at position 112 respectively correspond to the glutamic acid residue at position 60 and the glutamic acid residue at position 73 in the literatures.

Additionally, amino acid residues involved in the binding between barnase and barstar are the arginine residue at position 122, the arginine residue at position 126, and the histidine residue at position 141 in the amino acid sequence specified under GenBank ACCESSION No: AAA86441.1. There are reports that when these amino acid residues were mutated, the binding activity between barnase and barstar was reduced or lost (see Gideon Schreiber and Alan R. Fersht, J. Mol. Biol., 1995, vol. 248, pp. 478 to 486, Gideon Schreiber et al., Structure, October 1, 1994, vol. 2, iss. 10, pp. 945 to 951, Gideon Schreiber, Nuclease Methods and Protocols, Methods in Molecular Biology, 2001, vol. 160, pp. 213 to 226).

Note that the arginine residue at position 122, the arginine residue at position 126, and the histidine residue at position 141 respectively correspond to the arginine residue at position 83, the arginine residue at position 87, and the histidine residue at position 102 in the literatures.

Further, numerous reports have been made on the results of the structural analyses on barnase and barnase-substrate complexes (as to the X-ray crystal structure analysis on barnase, see "Martin C. et al., Acta Crystallographica D Biological Crystallography, 1999, vol. 55, pp. 386 to 398. As to the NMR analysis on barnase, see Bycroft M. et al . , Biochemistry, vol. 30, pp. 8697 to 8701. As to the X-ray crystal structure analysis on barstar, see Guillet V. et al., Proteins, 1993, vol. 17, pp. 325 to 328. As to the X-ray crystal structure analysis on a complex between barnase and a substrate analog guanosine 3'-phosphate, see Guillet V. et al., FEBS Letters, 1993, vol. 330, pp. 137 to 140. As to the X-ray crystal structure analysis on a complex between barnase and a substrate analog tetradeoxynucleotide CGAC, Buckle M. and Fersht A., Biochemistry, 1994, vol. 33, pp. 1644 to 1653. As to the X-ray crystal structure analysis on barnase-barstar complexes, see Guillet V. et al., Structure, vol. 1, pp. 165 to 177, and Buckle M. et al. Biochemistry, 1994, vol. 33, pp. 8878 to 8889).

Moreover, there is also a report that the active center of barnase is the histidine residue at position 141 and the glutamic acid residue at position 112 in the amino acid sequence specified under GenBank ACCESSION No: AAA86441.1 based on these structural analysis results (see Mossakowska et al. Biochemistry, 1989, vol. 28, pp. 3843 to 3850). Further, it has also been revealed that the serine residue at position 96, the asparagine residue at position 97, the arginine residue at position 98, and the glutamic acid residue at position 99 in the amino acid sequence specified under GenBank ACCESSION No: AAA86441. are involved in the binding to a guanine base in a substrate RNA molecule, and that the lysine residue at position 66, the arginine residue at position 122, the arginine residue at position 126, and the histidine residue at position 141 in the amino acid sequence are involved in the binding to a phosphate group in an RNA molecule. Furthermore, it has also been revealed that, among these amino acid residues in the amino acid sequence specified under GenBank ACCESSION No:AAA86441.1, if each of the lysine residue at position 66, the glutamic acid residue at position 112, and the histidine residue at position 141 is substituted with another amino acid residue, the enzymatic activity of barnase is greatly reduced.

Thus, the "barnase" according to the present invention preferably has no mutation introduced in the lysine residue at position 66, the serine residue at position 96, the asparagine residue at position 97, the arginine residue at position 98, the glutamic acid residue at position 99, the glutamic acid residue at position 112, the arginine residue at position 122, the arginine residue at position 126, and the histidine residue at position 141 in the amino acid sequence specified under GenBank ACCESSION No: AAA86441.1.

Further, whether or not the RNase activity of a barnase mutant is substantially equivalent to the activity of the wildtype barnase can be examined, for example, according to the method described in Gideon Schreiber et al. , J. Mol . Biol., 1997, vol. 270, pp. 111 to 122. Specifically, if a rate of degrading RNA (for example, yeast total RNA and tRNA) by a certain amount of a barnase mutant is generally 5 to 200, preferably 10 to 150, and more preferably 30 to 120, provided that the degradation rate by the wildtype barnase is taken as 100, it can be determined that the RNase activity of the examined barnase mutant is substantially equivalent to that of the wildtype barnase.

Furthermore, regarding whether or not the binding ability to barstar of a barnase mutant is substantially equivalent to that of the wildtype barnase, it can be determined that the binding ability to barstar of the examined barnase mutant is substantially equivalent to that of the wildtype barnase, if preferably 0.001 pM to 10 pM (0. 1 to 1000 times as large as a dissociation constant (0.01 pM) of the wildtype barnase from wildtype barstar to be described later), more preferably 0.001 pM to 0.1 pM (0. 1 to 10 times as large as the dissociation constant (0.01 pM) of the wildtype barnase from the wildtype barstar to be described later), for example, Gideon Schreiber et al . , J. Mol.

In addition, it has been expected that the plant cells are highly likely to die even if the "transit signal peptide to the organelles" is added to the "function inhibiting factor of the organelles" according to the present invention because the synthesis itself of the fusion protein of these takes place in the cytoplasm. However, surprisingly, it has been found out as described in Examples later that the addition of the "transit signal peptide to the organelles" to the "function inhibiting factor of the organelles" allows specific destruction of only the organelles while avoiding the death of the plant cells themselves.

Thus, it is only necessary that the "transit signal peptide to the organelles" according to the present invention be synthesized in a form fused with the function inhibiting factor in the cytoplasm of a plant cell or the like so as to have a signal for translocating the function inhibiting factor to plastids or mitochondria. Examples of the signal peptide include a chloroplast transit signal peptide of a ribulose bisphosphate carboxylase small subunit (RbcS) (see Niwa Y et al., The Plant Journal, 1999, vol. 18, iss. 4, pp. 455 to 463), a chloroplast transit signal of CRR4 (see Okuda et al., The Journal of biological chemistry, 2006, vol. 281, no. 49, pp. 37661 to 37667), a chloroplast transit signal of AtTadA (see Karcher and Bock, RNA, 2009, vol. 15, pp. 1251 to 1257), amitochondrion transit signal peptide of a mitochondrial RNA editing factor (MEF1) gene (see Zehrmann A et al., The Plant Cell, February 2009, vol. 21, pp. 558 to 567), and the like.

The form of the transit signal peptide fused with the function inhibiting factor is not particularly limited, as long as the transit signal functions. The transit signal peptide may be fused on either the N-terminus side or the C-terminus side of the function inhibiting factor. Furthermore, the transit signal peptide may be fused therewith directly or indirectly via a linker. Such a linker peptide has a length of generally 1 to 100 amino acids, preferably 1 to 30 amino acids.

Moreover, in the present invention, the "fusion protein containing the function inhibiting factor of the organelles and the transit signal peptide to the organelles" may be fused with another functional protein. In this case, the other functional protein may be fused on one or both of the N-terminus side and the C-terminus side of the fusion protein, or can be fused directly or indirectly between the function inhibiting factor and the transit signal peptide. The other functional protein is not particularly limited, and selected as appropriate depending on a function provided to the fusion protein according to the present invention. If the function to be provided to the fusion protein is for example to purify or detect the fusion protein or other similar functions, examples of the other functional protein include a green fluorescent protein (GFP), a luciferase protein, a Myc-tag (tag) protein, a His-tag protein, a hemagglutin (HA)-tag protein, a FLAG-tag protein (registered trademark, Sigma-Aldrich Co.), and a glutathione S-transferase (GST) protein.

In the present invention, the method for expressing the fusion protein in the plant cells is not particularly limited, and known techniques may be selected for use as appropriate. An example of such known techniques includes a method for introducing into plant cells a "vector comprising
a promoter capable of functioning in the plant cells, and
a DNA operably linked to the promoter and encoding the fusion protein (hereinafter, may also be referred to as "intracellular expression vector")" to be described later.

Note that the "plant cells" according to the present invention include, besides culture cells, cells in a plant. Further, the plant cells include plant cells in various forms, for example, suspended culture cells, protoplasts, leaf segments, calli, immature embryos, pollens, and the like.

Moreover, the fusion protein may be expressed in the plant cells transiently or constitutively, or the expression may be induced in response to a stimulus. Nevertheless, from the viewpoint that the expression of the fusion gene influences the growth of a plant composed of the plant cells by inhibiting the function of the organelles, the expression of the function inhibiting factor is preferably induced in response to a stimulus. Examples of such a stimulus include growth in the presence or absence of certain compounds, infection and invasion of filamentous fungi, bacteria, and viruses, low temperature, high temperature, dryness, and ultraviolet irradiation.

It can be recognized that, under these stimuli, the plant and the culture cells may receive an unintentional stimulus from the environment, and that although at low level, the target gene is expressed non-inducibly to some extent. Nevertheless, in a case of certain chemicals that are not generally present in the environment, the plant and the culture cells do not receive an unintentional stimulus. Particularly, the plant and the culture cells are preferably grown in the presence of estradiol, tetracycline, dexamethasone, tebufenozide, methoxyfenozide, ethanol, or copper because there are many findings regarding the induction stability and the non-inducible expression occurs at low level.

Note that when dicots are targeted by the present invention, the expression of the function inhibiting factor can be induced by estradiol according to the present invention, using the vector and the method described in Zuo J et al., Plant J, 2000, vol. 24, pp. 265 to 273. Meanwhile, when monocots are targeted by the present invention, the expression can be induced using the vector and the method described in Okuzaki A, Plant Cell Rep, 2011, vol. 30, pp. 529 to 538.

Moreover, in the case where the fusion protein is constitutively expressed in the plant cells or the expression is induced in response to a stimulus, the DNA encoding the fusion protein is inserted in the nuclear genomic DNA of the plant cells. In such a case, the number of copies of the gene (the DNA encoding the fusion protein) inserted in the nuclear genomic DNA is preferably small (1 to 5), more preferably one copy. Even in the case where the expression is induced in response to a stimulus, an expression independent of the stimulus occurs at low level, and even such a low-level expression inhibits the plant growth in some cases. Accordingly, the larger the number of copies, the more likely that the level of expression independent of the stimulus is increased. Additionally, if the number of copies is large, there is another trend that the expression of the fusion protein in the plant cells is likely to be suppressed due to silencing. Hence, the number of copies is preferably small as described above.

Moreover, it has been verified as described in Examples later that even when a heterozygote is produced by inserting one copy of the DNA encoding the fusion protein into the nuclear genomic DNA, the heterozygote sufficiently functions. Accordingly, it has been revealed that a plant, in which the DNA encoding the fusion protein is introduced in the nuclear genomic DNA, can be utilized in the above-described transformation of the organelles having an own genomic DNA in a plant. Thus, from the viewpoint of reducing the time and effort in transforming the organelles, and also from the viewpoint of a trend that the level of expression independent of the stimulus is increased when a homozygote is produced, the plant, in which the DNA encoding the fusion protein is introduced into the nuclear genomic DNA, is preferably heterozygous, more preferably heterozygous with one copy of the DNA encoding the fusion protein being inserted in the nuclear genomic DNA.

Meanwhile, as the method for introducing the vector of the present invention into plant cells, various methods known to those skilled in the art may be employed, for example, a polyethylene glycol method, an electroporation method, an *Agrobacterium*-mediated method, a particle gun method, a method using laser ablation, a whisker method, and the like.

The "restoring factor of the organelles" according to the present invention means a factor capable of directly or indirectly suppressing the inhibition of the function of the organelles by the function inhibiting factor. An example of such a restoring factor includes barstar in a case where barnase is used as the function inhibiting factor. Further, the examples include substances capable of binding to the function inhibiting factor to thereby inhibit the function of the factor, more specifically, antibodies (single-chain antibodies, nano-antibodies) against the function inhibiting factor, nucleic acid aptamers, and peptide aptamers.

Note that the "barstar" according to the present invention is an intracellular inhibiting factor derived from *Bacillus amyloliquefaciens* and capable of specifically binding to barnase, and is typically a protein (a protein having an amino acid sequence specified under GenBank ACCESSION No: CAA33551.1) (hereinafter, may also be referred to as "wildtype barstar") encoded by a DNA sequence specified under GenBank ACCESSION No: X15545.

Moreover, the amino acid sequence of the "barstar" according to the present invention may be mutated naturally (i.e., non-artificially). Meanwhile, a mutation can also be introduced artificially. Thus, the present invention comprises such mutants also, as long as the binding ability to barnase is substantially equivalent to that of the wildtype barstar.

The barstar mutants include proteins having an amino acid sequence specified under GenBank ACCESSION No: CAA33551.1, in which one or more amino acids are substituted, deleted, added, and/or inserted. Here, the term "more" refers to generally 20 amino acids or less, preferably 10 amino acids or less, more preferably several amino acids or less (for example, 5 amino acids or less, 3 amino acids or less, 2 amino acids or less, 1 amino acid).

Further, there is a report that, in barstar, the aspartic acid residue at position 39 in the amino acid sequence specified under GenBank ACCESSION No: CAA33551.1 is important for binding to barstar (see Gideon Schreiber et al., J. Mol. Biol., 1997, vol. 270, pp. 111 to 122).

Note that, herein, each amino acid residue in the amino acid sequence specified under GenBank ACCESSION No: CAA33551.1 is specified with the first methionine residue (the methionine residue encoded by the start codon) being position 0.

Furthermore, amino acid residues involved in the binding between barnase and barstar are the aspartic acid residue at position 35 and the aspartic acid residue at position 39 in the amino acid sequence specified under GenBank ACCESSION No: CAA33551.1. There are reports that when these amino acid residues were mutated, the binding activity between barnase and barstar was reduced or lost (see Gideon Schreiber and Alan R. Fersht, J. Mol. Biol., 1995, vol. 248, pp. 478 to 486, Gideon Schreiber et al., Structure, October 1, 1994, vol. 2, iss. 10, pp. 945 to 951, Gideon Schreiber, Nuclease Methods and Protocols, Methods in Molecular Biology, 2001, vol. 160, pp. 213 to 226) .

Thus, the "barstar" according to the present invention preferably has no mutation introduced in these amino acid residues.

Moreover, regarding whether or not the binding ability to the wildtype barnase of a barstar mutant is substantially equivalent to that of the wildtype barstar, it can be determined that the binding ability to the wildtype barnase of the examined barstar mutant is substantially equivalent to that of the wildtype barstar, for example, if a dissociation constant between the barstar mutant and barnase obtained according to the method described in Gideon Schreiber et al., J. Mol. Biol., 1997, vol. 270, pp. 111 to 122 is generally 0.001 pM to 1 nM (0.1 to 100,000 times as large as the dissociation constant (0.01 pM) of the wildtype barnase from the wildtype barstar to be described later), preferably 0. 001 pM to 10 pM (0.1 to 1000 times as large as the dissociation constant (0.01 pM) of the wildtype barnase from the wildtype barstar to be described later), more preferably 0.001 pM to 0.1 pM (0. 1 to 10 times as large as the dissociation constant (0.01 pM) of the wildtype barnase from the wildtype barstar to be described later).

In the present invention, the method for introducing into the genomic DNA of the organelles of the plant cells the "expression cassette comprising the DNA encoding the restoring factor and the DNA encoding the factor desired to be expressed in the organelles is not particularly limited, and known techniques may be selected for use as appropriate. An example of such known techniques includes a method for introducing into plant cells a "vector comprising
an expression cassette comprising a first promoter capable of functioning in the organelles, the DNA operably linked to the first promoter and encoding the restoring factor, a second promoter capable of functioning in the organelles, and the DNA operably linked to the second promoter and encoding the factor desired to be expressed in the organelles, and

DNA sequences located on both sides of the expression cassette and having a homology with the genomic DNA specific to the organelles (hereinafter, may also be referred to as "organelle transformation vector")" to be described later.

Note that, as the method for introducing the vector of the present invention into plant cells, various methods known to those skilled in the art may be employed, for example, a polyethylene glycol method (see O'Neill C et al., The Plant Journal, 1993, vol. 3, iss. 5, pp. 729 to 738), an electroporation method (see Shimogawara Ket al., Genetics, April 1998, vol. 148, pp. 1821 to 1828), an Agrobacterium-mediated method (see Block De M et al. , The EMBO Journal, 1985, vol. 4, iss. 6, pp. 1367 to 1372), a laser ablation method (for example, a method for introducing into organelles of plant cells gold particles coated with an organelle transformation vector using a laser, see Kajiyama S et al., Journal of Bioscience and Bioengineering, 2008, vol. 106, iss. 2, pp. 194 to 198), a particle gun method, a whisker method (see Nagatani N et al., Biotechnol Tech., 1997, vol. 11, pp. 471 to 473, Frame BR et al. , Plant J. , 1994, vol. 6, pp. 941 to 948), and the like.

Moreover, in a case where the genes are introduced into plastids (chloroplasts) of, for example, potato, it can be done with reference to the method described in Sidorov et al., The Plant Journal, 1999, vol. 19, iss. 2, pp. 209 to 216. In a case of tomato, the genes can be introduced, for example, with reference to the method described in Ruf et al., Nature Biotechnology, 2001, vol. 19, pp. 870 to 875 (NPL 3) . In a case of canola, the genes canbe introduced with reference to the method described in Hou et al., Transgenic Research, 2003, vol. 12, pp. 111 to 114 . Further, in a case of *Lesquerella fendleri,* the genes can be introduced with reference to the method described in Skarjinskaia et al., Transgenic Research, 2003, vol. 12, pp. 115 to 122, and in a case of petunia, the genes can be introduced with reference to the method described in Zubko et al., Transgenic Research, 2004, vol. 13, pp. 523 to 530. Additionally, in a case of soybean, the genes can be introduced with reference to the method described in Dufourmantel et al., Plant Molecular Biology, 2004, vol. 55, pp. 479 to 489; in a case of carrot, the genes can be introduced with reference to the method described in Kumar et al. , Plant Physiology, 2004, vol. 136, pp. 2843 to 2854; and in a case of cotton, the genes can be introduced with reference to the method described in Kumar et al., Plant Molecular Biology, 2004, vol. 56, pp. 203 to 216. Furthermore, in a case of poplar, the genes can be introduced with reference to the method described in Okumura et al. , Transgenic Res, 2006, vol. 15, pp. 637 to 646; in a case of lettuce, the genes can be introduced with reference to the method described in Lelivelt et al., Plant Molecular Biology, 2005, vol. 58, pp. 763 to 774; and in a case of *Arabidopsis thaliana,* the genes can be introduced with reference to the method described in Sikdar et al. , Plant Cell Reports, 1998, vol. 18, pp. 20 to 24. Moreover, in a case of cauliflower, the genes can be introduced with reference to the method described in Nugent et al., Plant Science, 2006, vol. 170, pp. 135 to 142; in a case of cabbage, the genes can be introduced with reference to the method described in Liu et al., Plant Cell Rep, 2007, vol. 26, pp. 1733 to 1744; and in a case of sugar beet, the genes can be introduced with reference to the method described in De Marchis et al., Transgenic Res, 2009, vol. 18, pp. 17 to 30. Further, in a case of tobacco (*Nicotiana benthamiana*), the genes can be introduced with reference to the method described in Davarpanah et al . , J. Plant Biol . , 2009, vol. 52, pp. 244 to 250; in a case of eggplant, the genes can be introduced with reference to the method described in Singh et al, Transgenic Res, 2010, vol. 19, pp. 113 to 119 (NPL 6); and in a case of rice, the genes can be introduced with reference to the method described in Lee et al. , Mol. Cells, 2006, vol. 21, iss. 3, pp. 401 to 410 (NPL 9).

The expression cassette introduced in the organelles of the plant cells by these methods is then introduced into the genomic DNA of the organelles by homologous recombination that occurs between the genomic DNA specific to the organelles and the DNA sequences located on both sides of the expression cassette.

Although the description has been given of each step in the transformation method of the present invention, the method can be performed more specifically as follows.

For example, in a case where the transformation method of the present invention is employed to dicots such as tobacco, first, the intracellular expression vector according to the present invention is introduced into plant cells (for example, plant cells in a leaf) of the plant by the above-described known methods. Then, calli from the plant cells (or adventitious shoots formed from the calli) are prepared, followed by selection of calli or the like having the vector introduced in the genomic DNA. Note that such selection can be made using known DNA detection techniques (for example, PCR, Southern blotting, sequencing) . Next, from the calli or the like thus obtained, plant individuals are regenerated. Thereafter, the organelle transformation vector according to the present invention is introduced into plant cells (for example, leaf segments) of the individuals by the known methods. Subsequently, calli or the like from the plant cells are prepared, followed by selection of calli or the like having the vector introduced in the genomic DNA of the organelles using the known DNA detection techniques.

After that, in plant cells of calli or the like thus obtained, the organelles, in which the organelle transformation vector is not introduced, are positively destroyed by the function inhibiting factor as described in Examples later. This increases a proportion of the organelles expressing the factor desired to be expressed.

Moreover, even if homoplasmic plant individuals or highly homoplasmic plant individuals are not obtained in the above-described method, homoplasmic plant individuals or highly homoplasmic plant individuals can be obtained efficiently according to the method of the present invention as described in Examples later by preparing calli or the like again from heteroplasmic individuals (plants composed of heteroplasmic plant cells or chimeric plants), and by regenerating plant individuals from the calli or the like.

Further, as described later, the selection can be made by using an organelle transformation vector comprising a selection marker such as a chemical-resistance gene in such a manner that calli or the like having the vector introduced in the genomic DNA of the organelles are cultured under a selection pressure (for example, cultured on a medium supplemented with a chemical or the like).

Furthermore, in the case where the expression of the function inhibiting factor is induced by a stimulus, it is particularly preferable to use an organelle transformation vector comprising a selection marker. In the very initial period when the organelle transformation vector is introduced, the number of chloroplasts expressing the restoring factor is too small. Accordingly, when the expression of the function inhibiting factor is induced by a stimulus during the period, wildtype chloroplasts accounting for most of chloroplasts in plant cells are disrupted, making it difficult to grow even plant cells having the organelle transformation vector introduced. For this reason, it is particularly preferable to use the organelle transformation vector comprising the selection marker in such a manner that plant cells having the vector introduced are first cultured under a selection pressure corresponding to the selection marker, and then the expression of the function inhibiting factor is induced in response to a stimulus.

Thus, a preferable embodiment of the transformation method of the present invention also includes the following.

A method for transforming organelles having an own genomic DNA in a plant, the method comprising the steps of:
introducing into the genomic DNA of the organelles of plant cells an expression cassette comprising a DNA encoding a restoring factor of the organelles, a DNA encoding a factor desired to be expressed in the organelles, and a DNA encoding a selection marker;
culturing the plant cells under a selection pressure corresponding to the selection marker;
after the culturing, inducing an expression of a fusion protein containing a function inhibiting factor of the organelles and a transit signal peptide to the organelles in the plant cells in response to a stimulus; and
allowing the function inhibiting factor to destroy the organelles, in which the expression cassette is not introduced, in the plant cells.

The "period during which the plant cells are cultured under the selection pressure corresponding to the selection marker" is preferably 3 to 20 weeks, more preferably 4 to 8 weeks. If the period is shorter than the lower limit, there is a trend that the growth of the cells containing the transformed organelles is not sufficient to form a tissue, making it difficult to form re-differentiated adventitious shoots . If the period exceeds the upper limit, there is a trend that the re-differentiation ability of an explant used is reduced. Besides, undesirable traits may be exhibited, such as a high possibility of obtaining abnormal plants and male sterile plants due to somaclonal variation. Furthermore, there is a trend that re-differentiated adventitious shoots are likely to be obtained from non-chloroplast transformants resistant to the selection pressure due to a mutation in an endogenous gene, depending on the selection marker.

Meanwhile, the selection pressure includes additions to a medium of chemicals such as spectinomycin, hygromycin, kanamycin, and herbicides. Further, those skilled in the art can adjust the concentration of such a chemical added to a medium as appropriate by taking the type of the chemical used and the expression level of the selection marker into consideration.

Note that, in selecting plant cells, in which the DNA encoding the factor desired to be expressed in the organelles is introduced, selections using chemicals such as antibiotics and herbicides have been conventionally practiced. However, such selections with chemicals alone cannot successfully select plant cells having the DNA introduced, and there are plant species from which only heteroplasmic or chimeric transformants are obtained. In addition, even plant species from which homoplasmic transformants are obtained by such a method require a long-term selection period.

Hence, a two-stage selection, in which selection using a chemical or the like is combined with a selection utilizing the function inhibiting factor and the restoring factor as described above, makes it possible to promote homoplasmy in any plant. Furthermore, the two-stage selection makes it possible to increase a proportion of the transformed plastids or the like in plant species, which have quite a low efficiency of transforming plastids and the like, so that only plant cells and the like containing few transformed plastids and the like are obtained, and which allow recombinant DNA detection just by a PCR analysis.

Meanwhile, to regenerate plant individuals from calli or the like, ordinary methods in the technical field can be employed. Examples of such ordinary methods include methods described in "Nagata et al., Planta, 1971, vol. 99, pp. 12 to 20, " "Akama et al., Plant Cell Reports, 1992, vol. 12, pp. 7 to 11, " "Visser et al. , Theor. Appl . Genet. , 1989, vol. 78, pp. 594 to 600," "Shillito et al., Bio/Technology, 1989, vol. 7, pp. 581 to 587, " "Gorden-Kamm et al., Plant Cell, 1990, vol. 2, pp. 603 to 618."

Further, in a case, for example, where the transformation method of the present invention is employed to plants other than tobacco, the present invention can be performed according to the same method as those for tobacco and so on described above by selecting as target explants of the gene introduction: for Solanaceae plants, leaf segments as in the case of tobacco; for *Brassica* plants such as rapeseed, hypocotyls of aseptically-grown seedlings instead of leaf segments; and for Cucurbitaceae plants such as squash, basal parts of cotyledonary explants (as to squash and so on, see Nanasato Y et al. , Plant Cell Rep., August 2011, vol. 30, iss. 8, pp. 1455 to 1464). Furthermore, in cases of monocots, for example, the present invention can be performed according to the same method as those for tobacco and so on described above by selecting as target explants of the gene introduction: for rice, calli derived from seeds instead of leaf segments; and for corn and wheat-like plants, calli derived from immature embryos or calli derived from cotyledons instead of leaf segments (as to the method using calli derived from seeds of corn, see the method described in Ishida Y et al . , Nature Protocols, 2007, vol. 2, iss. 7, pp. 1614 to 1621 or the method described in Oneto et al . , Article Jouranl of Agricultural Research, 2010, vol. 5, no. 25, pp. 3561 to 3570. As to the cotyledon-derived callus method, see the method described in Ahmadabadi et al., Transgenic Res, 2007, vol. 16, pp. 437 to 448).

### <Transformation Vector>

The present invention provides at least one vector selected from the following (a) and (b) preferably used in the above-described transformation method.
(a) a vector comprising
   a promoter capable of functioning in the plant cells, and
   a DNA operably linked to the promoter and encoding the fusion protein; and
(b) a vector comprising
   an expression cassette comprising a first promoter capable of functioning in the organelles, the DNA operably linked to the first promoter and encoding the restoring factor, a second promoter capable of functioning in the organelles, and the DNA operably linked to the second promoter and encoding the factor desired to be expressed in the organelles, and
   DNA sequences located on both sides of the expression cassette and having a homology with the genomic DNA specific to the organelles.

In the vector described in (a) above of the present invention, the "promoter capable of functioning in the plant cells" is a promoter for constitutively or inducibly expressing the DNA encoding the fusion protein in the plant cells as described above.

Examples of the promoter for constitutive expression include a cauliflower mosaic virus 35S promoter, a rice actin promoter, and a corn ubiquitin promoter.

Meanwhile, examples of the promoter for inducible expression, that is, a promoter for inducing the expression in response to the stimulus, include a rice chitinase gene promoter and a tobacco PR protein gene promoter expressed by infection and invasion of filamentous fungi, bacteria, and viruses, a rice lip19 gene promoter induced by low temperature, rice hsp80 gene and hsp72 gene promoters induced by high temperature, an *Arabidopsis thaliana* rab16 gene promoter induced by dryness, a parsley chalcone synthase gene promoter induced by ultraviolet irradiation, and a corn alcohol dehydrogenase gene promoter induced by an anaerobic condition. In addition, the rice chitinase gene promoter and the tobacco PR protein gene promoter may be induced by a certain compound such as salicylic acid, and rab16 may be induced by a plant hormone, abscisic acid. Further, the examples also include an alc promoter induced by ethanol and a tetracycline-responsive promoter induced by tetracycline. Moreover, as described in Examples later, the example also includes a promoter for constitutively expressing in cells a protein, in which a certain chemical receptor, a certain transcription activation domain, and so forth are fused together, the fusion protein being translocated to the nucleus when the fusion protein receives the chemical, the promoter being capable of inducing the expression of a gene located downstream by binding with the transcription activation domain . Examples of such a promoter include the following promoters.

A promoter containing an LexA responsive sequence, which is activated by binding of estradiol and a synthetic transcription activating factor XVE, in which a partial sequence of a repressor LexA of an *Escherichia coli* SOS regulon, a transcription activation domain of HSV (herpes simplex virus)-derived VP16, and a regulatory region of an estrogen receptor are fused together (see (A) in Fig. 2) ;
a promoter containing an ER responsive sequence, which is activated by binding of estradiol and a synthetic transcription activating factor, in which a transcription activation domain of C1 and an estrogen receptor are fused together (see Bruce W. et al., Plant Cell, 2000, vol. 12, pp. 65 to 80);
a promoter containing a GAL4 responsive sequence, which is activated by binding of estradiol and a synthetic transcription activating factor, in which a DNA-binding site of GAL4, a transcription activation domain of VP16, and a ligand-binding site of an estrogen receptor are fused together (see Zhang S., Novartis Found Symp, 2001, vol. 236, pp. 85 to 96);
a promoter containing a GAL4 responsive sequence, which is activated by binding of dexamethasone and a synthetic transcription activating factor, in which a DNA-binding site of GAL4 , a transcription activation domain of VP16, and a ligand-binding site of a glucocorticoid receptor are fused together, (see Aoyama T. et al., Plant J, 1997, vol. 11, pp. 605 to 612);
a promoter containing a GAL4 responsive sequence, which is activated by binding, in the absence of tetracycline, of dexamethasone and a synthetic transcription activating factor, in which a tetracycline repressor, a transcription activation domain of VP16, and a ligand-binding site of a glucocorticoid receptor are fused together (see Bohner S. et al. , Plant J, 1999, vol. 19, pp. 87 to 95);
a promoter containing a glucocorticoid receptor responsive sequence, which is activated by binding of tebufenozide and a synthetic transcription activating factor, in which a DNA-binding site of a glucocorticoid receptor, a transcription activation domain of VP16, and a ligand-binding site of a *Heliothies virescens* ecdysone receptor, are fused together (see Martinez A. et al. , Plant J, 1999, vol. 19, pp. 97 to 106);
a promoter containing a GAL4 responsive sequence, which is activated by binding of methoxyfenozide and a synthetic transcription activating factor, in which a DNA-binding site of GAL4, a transcription activation domain of VP16, and a *Choristoneura fumiferana* ecdysone receptor are fused together (see Padidam M. et al., "Chemical-inducible, ecdyson receptor-based gene expression system for planets. ", Transgenic Res, 2003, viol. 12); and
a promoter containing a GAL4 responsive sequence, which is activated by binding of methoxyfenozide and a synthetic transcription activating factor, in which a DNA-binding site of GAL4 , a transcription activation domain of C1, and an *Ostrinia nubilalis* ecdysone receptor are fused together (see Unger E., Transgenic Res, 2002, vol. 11, pp. 455 to 465).

Further, as apparent from the descriptions of these promoter examples, those skilled in the art can construct a system for inducibly expressing the DNA encoding the fusion protein in plant cells by selecting an appropriate combination of a certain chemical, a receptor corresponding thereto, a transcription activation site, a DNA-binding site, and a promoter containing a responsive sequence correspondingtotheDNA-bindingsite. Furthermore, based on the DNA expression or the like in such a system, those skilled in the art can prepare an amino acid-substituted synthetic transcription activating factor and a base-substituted promoter also by using known techniques as appropriate.

In the vector described in (b) above of the present invention, the "first and second promoters capable of functioning in the organelles" are promoters for expressing specifically in the organelles the DNA encoding the restoring factor or the DNA encoding the factor desired to be expressed in the organelles.

Examples of such promoters includes, in a case where the organelles are plastids, a chloroplast-derived rRNA operon promoter (Prrn2), a chloroplast-derived photosynthesis-related gene psbA promoter (PpsbA), a chloroplast-derived photosynthesis-related gene rbcL promoter, and a psaA promoter. In a case where the organelles are mitochondria, the examples include an ATP9 promoter, a rrn26 promoter, and a rrn18 promoter.

Note that the first promoter and the second promoter may be the same type of promoters (for example, the first promoter and the second promoter are both Prrn2), or the first promoter and the second promoter may be different types of promoters (forexample, the first promoter is Prrn2, whereas the second promoter is PpsbA) . Moreover, the first promoter may be different from or the same as the second promoter. Specifically, in the organelle, multiple genes are linked to one promoter, and one terminator sequence is further linked thereto to polycistronically express mRNA, from which corresponding single proteins can be synthesized at the translation stage (see Arai Y et al., Plant Cell Physiol., 2004, vol. 45, no. 9, pp. 1176 to 1184). Accordingly, multiple genes (the gene encoding the restoring factor, the gene encoding the factor desired to be expressed in the organelles, a selection marker to be described later, and so forth) may be expressed by single promoter (the first promoter or the second promoter) and terminator.

Further, in a case where the expression cassette comprises multiple DNAs encoding the factor desired to be expressed, the second promoter may be operably linked to each of the multiple DNAs. In this case, the multiple second promoters may be the same type of promoters, or may be different types of promoters from each other. Furthermore, the second promoter may be a single promoter so that the multiple DNAs encoding the factor desired to be expressed can be expressed as one polycistronic mRNA as described above.

In the vector described in (b) above of the present invention, examples of the "DNA sequences having a homology with the genomic DNA specific to the organelles" include, in the case where the organelles are plastids, DNA sequences having a homology with a chloroplast genomic DNA containing chloroplast trnI gene, trnA gene, rbcL gene, or accD. In the case where the organelles are mitochondria, the examples include DNA sequences having a homology with a mitochondrial genomic DNA containing rrn18 or rrn26. In addition, the "DNA sequences having a homology with the genomic DNA specific to the organelles" are preferably DNA sequences having a homology with the genomic DNA containing a region, in which a site where the expression cassette is inserted is not a gene coding region, and more preferably DNA sequences having a homology with the genomic DNA of within approximately 1000 to 4000 bases from the insertion site.

Moreover, the vector of the present invention may comprise a selection marker, a replication origin, a terminator, a polylinker, an enhancer, a ribosomal binding site, and so forth as appropriate. Generally, the DNA encoding the fusion protein and so forth are located downstream of the promoter, and further a terminator is located downstream of the gene.

Examples of such a terminator include a psbA gene terminator (TpsbA), a chloroplast-derived ribosomal protein S16 terminator (Trps16), a chloroplast-derived rps16 gene terminator (TrbcL), a mitochondrion-derived ATP9 terminator, a rrn26 terminator, a rrn18 terminator, a cauliflower mosaic virus-derived terminator, and a nopaline synthase gene-derived terminator.

Further, the use of the vector comprising a selection marker enables selection regarding whether or not the vector of the present invention is introduced in plant cells, by adding a chemical or the like as described in Examples later. Examples of such a selection marker includes a hygromycin-resistance gene (HPT), a spectinomycin-resistance gene (aadA), a kanamycin-resistance gene (NPTII), herbicide-resistance genes (a bialaphos resistance gene (bar), an acetolactate synthase-inhibiting herbicide-resistance gene (mALS)), and the like.

### <Plant Transformant>

The present invention provides a transformant produced by the above-described transformation method. Moreover, the present invention also provides a transformant comprising at least one DNA selected from the following (a) and (b):
(a) a promoter capable of functioning in the plant cells and a DNA operably linked to the promoter and encoding the fusion protein; and
(b) a first promoter capable of functioning in organelles having an own genomic DNA in a plant, a DNA operably linked to the first promoter and encoding the restoring factor, a second promoter capable of functioning in the organelles, and a DNA operably linked to the second promoter and encoding the factor desired to be expressed in the organelles.

Note that the DNA in (b) encodes the promoters capable of functioning in the organelles and the factor desired to be expressed. Thus, the DNA in (b) is to be introduced into the genomic DNA of the organelles in the transformant of the present invention.

Moreover, the transformation method of the present invention enables efficiently production of a highly homoplasmic plant composed mostly of highly homoplasmic plant cells, in which most of the organelles are transformed. Once such a highly homoplasmic plant is obtained, it is possible to obtain a progeny or a clone from the plant by sexual reproduction or asexual reproduction. Specifically, once a highly homoplasmic plant is obtained, even if barnase and so forth are genetically separated and lost in offspring individuals produced by crossing or the like, highly homoplasmic plant progenies can be obtained by producing highly homoplasmic offspring individuals through self-fertilization or crossing using highly homoplasmic individuals as a female parent because the homoplasmy of the offspring individuals is maintained. In addition, clones of the highly homoplasmic plant can be obtained by cutting a highly homoplasmic individual, aseptically culturing a leaf or root of the highly homoplasmic individual, and growing plants that have dedifferentiated and then re-differentiated.

Further, the present invention can provides, as the transformant of the present invention, not only the plant, but also a propagation material (for example, a seed, a fruit, a spike, a stem tuber, a tuberous root, a callus, a protoplast, and the like) obtained from any one of a progeny and a clone of the plant; and a plant cell, a plant tissue (for example, an epidermis, a vascular bundle), and a plant organ (for example, a leave, a stem, a root) composing the plant.

Furthermore, the present invention provides a processed product of the transformant, comprising an expression product of the factor desired to be expressed in the organelles.

Such processed products refer to processed products in general, which have been conventionally produced from plants. Examples thereof include liquid extracts from plants, and plant dried powders and processed foods. Moreover, such processed foods includes, in a case of rice, cooked rice, rice crackers, and the like; in a case of wheat, breads, noodles, and the like; in a case of corn, corn oil, corn starch, corn chips, and the like; in a case of soybean, soybean oil, tofu, natto, and the like; in a case of potato, potato chips, starches, and the like; in a case of tomato, ketchup and the like; and in a case of canola, canola oil and the like.

### [Examples]

Hereinafter, the present invention will be described more specifically based on Examples. However, the present invention is not limited to the following Examples.

### (Example 1)

### <Preparation of Barnase Inducing Expression Binary Vector pNtLextpBn, and Introduction into Agrobacterium>

In order to induce the expression of barnase by estradiol and to then translocate barnase to a chloroplast and function therein, pNtLextpBn was designed and prepared as follows according to the description of "Zuo J et al., Plant J, 2000, vol. 24, pp. 265 to 273."

First, PCR was carried out using a plasmid vector pBarnase having a *Bacillus amyloliquefaciens-derived* barnase gene sequence as a template, and a primer bnF-SalI (5`-GTGTCGACGCACAGGTTATCAACACGTT-3', SEQ I D NO: 1) and a primer bnR-PacI (5'-GTTTAATTAATTATCTGATTTTTGTAAAGGTC-3', SEQ ID NO: 2). The amplified fragment thus obtained was inserted and cloned in a pCR2.1-TOPO vector having a XhoI site at a site adjacent to the cloning site. Thus, pBnSP was obtained.

Moreover, in order to amplify a chloroplast transit signal sequence of *Arabidopsis thaliana-*derived RbcS-1A, an amplified fragment was obtained by a PCR method using a RbcS-1A-inserted plasmid vector as a template, and a primer AtTPF-XhoI 5'-AGCTCGAGATGGCTTCCTCTATGCTCTC-3' (the underline indicates a XhoI site. SEQ ID NO: 3) and a primer AttTPR-SalI 5'-TTGTCGACGCAGTTAACTCTTCCGCCGT-3' (the underline indicates a SalI site. SEQIDNO: 4) . Then, 0.17 kb of the resulting amplified fragment of the chloroplast transit signal sequence of RbcS-1A was digested with XhoI and SalI, and inserted in the XhoI/SalI site of pBnSP. Thus, pTPBarnase was obtained. Then, the pTPBarnase was digested with XhoI and PacI, and a fragment containing RbcS-1A-Barnase was inserted in the XhoI and PacI site downstream of a chemical-inducible promoter of an estradiol-inducible expression vector pER8 (see Zuo J et al., Plant J, 2000, vol. 24, pp. 265 to 273), which was obtained as pNtLextpBn (see A in Fig. 2). Subsequently, the pNtLextpBn was introduced into an *Agrobacterium* EHA105 strain (Hood EE et al., Transgenic Res, 1993, vol. 2, pp. 208 to 218), which was used for tobacco nuclear transformation.

### (Example 2)

### <Production of Barnase Gene-Introduced Tobacco (Nuclear Transformation)>

The barnase gene was introduced into the tobacco nucleus as follows.

First, tobacco (cultivar; SR1) seeds were subjected to a sterilization treatment for 10 minutes with a sterilization solution obtained by adding several drops of Tween 20 to a hypochlorous acid solution having an effective chlorine concentration of 0.5%. Then, after washing with sterile water, the sterilized seeds were seeded on a MS medium (see Murashige T and Skoog F, Physiol Plant, 1962, vol. 15, iss. 3, pp. 473 to 497), and grown for 16 hours in the light and in a incubator at 25°C for 4 weeks or longer.

Using leaves of the tobacco thus obtained as a material, the pNtLextpBn was introduced by an *Agrobacterium* method (see Horsch R. B. et al., Science, 1985, vol. 227, pp. 1229 to 1231). Subsequently, selection was made using a MSBN medium (re-differentiation medium obtained by adding 1.0 mgL⁻¹ of BA and 0.1 mgL⁻¹ of NAA to a MS medium) supplemented with 500 mgL⁻¹ of carbenicillin and 50 mgL⁻¹ of hygromycin. Thereafter, the selected hygromycin-resistant adventitious shoots were transplanted to a MS medium, and roots were developed to thereby produce hygromycin-resistant individuals. After that, in order to check that the gene had been introduced, the HPT gene and the barnase gene were amplified by a PCR analysis using as a template a DNA extracted from leaves of the hygromycin-resistant individuals. As a result, bands were observed from 14 individuals by the PCR analysis. Thus, barnase gene-introduced lines were obtained.

### (Example 3)

### <Inhibition of Chloroplast Function by Barnase Expression>

Next, from the barnase gene-introduced independent 14 lines, lines were selected, whose growth was inhibited by barnase expression specific to an estradiol treatment. Specifically, six leaf segments of 5 mm square were prepared and placed on a MSBN medium supplemented with 0 µM or 5.0 µM estradiol, followed by culturing for 20 days. As a control experiment, the same treatment was performed using wildtype leaf segments.

As a result, six barnase gene-introduced lines were inhibited from forming adventitious shoots from the leaf segments only when treated with 5.0 µM estradiol (see Fig. 3). Meanwhile, without estradiol, the adventitious shoot formation equivalent to that of the wildtype was observed in these six lines. This revealed that barnase expressed by the estradiol treatment was translocated to the chloroplasts, and inhibited the chloroplast function, consequently inhibiting the adventitious shoot re-differentiation. Hence, the lines were used in the subsequent experiments.

On the other hand, the other eight barnase gene-introduced lines were inhibited from forming adventitious shoots regardless of the estradiol treatment, suggesting that the non-inducible barnase expression was strong. It should be noted that adventitious shoot formation was observed in the wildtype leaf segments regardless of the estradiol treatment.

It has been assumed that the smaller the number of copies of the barnase gene inserted into the nucleus and chromosome of a line, the lower the non-inducible expression of the gene. Accordingly, a Southern blotting analysis was next performed on the six lines to examine the number of copies of the introduced gene.

Note that the Southern blotting was carried out as follows. First, 5 µg of a DNA extracted from each line was digested with HindIII, and the analysis was performed according to an ordinary method using a hpt probe. The hpt probe was prepared by amplification using hpt specific primers 5'-GAGCCTGACCTATTGCATCTC-3' (SEQ ID NO: 5) and 5'-GTACTTCTACACAGCCATCG-3' (SEQ ID NO: 6) with PCR DIG Labeling Mix (manufactured by Roche Diagnostics K. K.). Moreover, the detection was performed using DIG Luminescent Detection Kit (manufactured by Roche Diagnostics K. K.) according to the protocol.

As a result, it was revealed that one (#7 line) of the six lines was of individuals having one copy of the introduced gene. Hence, the #7 line, which was a line having one copy of the barnase gene introduced, was used to introduce a barstar gene to chloroplasts.

### (Example 4)

### <Preparation of Chloroplast Transformation Plasmid Vector pNtagBs Having Barstar Gene>

In order to introduce the barstar gene into chloroplasts, a plasmid vector pNtagBs was prepared as follows. Note that barstar is a protein that inhibits the activity of barnase.

First, PCR amplification was performed using a *Bacillus amyloliquefaciens*-derived barstar gene as a template, and a primer bsFBamHI (5'-GTCGGATCCATGAAAAAAGCAGTCATTAACG-3', SEQ ID NO: 7) and a primer bsRBamHI (5'-CCCGGATCCTTAAGAAAGTATGATGGTGATG-3', SEQ ID NO: 8). Then, the amplified fragment thus obtained was digested with BamHI, and inserted in a BamHI site downstream of a promoter of a plasmid pPrrn-TrbcL having a gene expression cassette for tobacco chloroplast transformation. Thus, pPrrn-Bs-TrbcL was prepared.

Next, in order to introduce the barstar gene between trnI and trnA genes of tobacco chloroplasts by homologous recombination, a sequence near a tobacco trnI-trnA region was cloned as a homologous region as follows. A trnI flanking region (tobacco chloroplast DNA, ACCESSION No. Z00044: 103417-105335, 1.9 kb) was amplified by PCR using a tobacco leaf DNA as a template, and NT-trnIF-SacII (5'-GTCCGCGGCGGAAAGAACACCAACGGCG-3', SEQ ID NO: 9) and NT-trnIR-NotI (5'-TCGCGGCCGCAGCTGGGCCATCCTGGACT-3', SEQ ID NO: 10). Then, the amplified fragment thus obtained was inserted and cloned in a pCR2.1-TOPO vector (manufactured by Invitrogen Corporation). Thus, pNttrnI was obtained.

Moreover, a trnA flanking region (tobacco chloroplast DNA, ACCESSION No. Z00044: 105330-106944, 1.6 kb) was amplified by PCR using a tobacco leaf DNA as a template, and NT-trnAF-SalI (5'-GAGTCGACAGCTGCGCCAGGGAAAAGA-3', SEQ ID NO: 11) and NT-trnAR-ApaI (5'-CCGGGCCCAAGCCACTGCCTATGAGTC-3', SEQ ID NO: 12). Then, the amplified fragment thus obtained was inserted and cloned in a pCR2.1-TOPO vector (manufactured by Invitrogen Corporation). Thus, ptrnA was obtained.

Further, a trnA fragment obtained by digesting the ptrnA with SalI and ApaI was inserted in the SalI/ApaI site of a pBluescriptII vector. Thus, pBtrnI was obtained. Subsequently, a trnI fragment cleaved from the pNttrnI with SacII and NotI was inserted in the SacII/NotI site of the pBtrnI. Thus, pBtrnI-trnA was prepared. Then, a sequence AP1 (5'-TCGACATCGATACGTACGTTAATTAACCTGCAGGCCCGGGCC-3', SEQ ID NO: 13) artificially synthesized to add a restriction enzyme site (NotI-BanIII-Eco105I-BsiWI-PacI-SrfI-SbfI-SalI) to the NotI and SalI site of the pBtrnI-trnA was inserted therein. Thus, a vector pNtAP1 was obtained.

Next, a cassette Prrn-T7G10-aadA-TpsbA-PpsbA-GFP-Trps16 capable of expressing an aadA gene as a selection marker (spectinomycin resistance) and a GFP gene as a visible marker was constructed. Specifically, first, aPrrn-T7G10 sequence (may also be referred to as "Prrn2" sequence) was constructed by adding a phage-derived T7G10 sequence (5'-GGGAGACCACAACGGTTTCCCTCTAGAAATAATTTTGTTTAACTTTAAGA AGGAGATATACAT-3', SEQ ID NO: 14) immediately downstream of a rrn promoter for aadA expression at high level. Note that the Prrn was prepared by PCR amplification using a tobacco chloroplast DNA as a template, and primers Prrn-F (5'-ATGCGGCCGCTTCTAGTTGGATTTGCTCCC-3', SEQ ID NO: 15) and Prrn-R (5'-AGTACGTAGACAAAGCGGATTCGGA-3', SEQ ID NO: 16). Moreover, the T7G10 was prepared by PCR amplification using pGEM Express Positive Control Template (manufactured by Promega Corporation) as a template, and primers T7G10-F (5'-ACCCGGGAGACCACAACGGTTTCCCT-3', SEQ ID NO: 17) and T7G10-R (5'-AGGATCCCATATGTATATCTCCTTCTTAAAG-3', SEQ ID NO: 18). Then, the full-length sequence of the aadA gene obtained by digesting pLD200-GFP (see Kajiyama S et al., Journal of Bioscience and Bioengineering, 2008, vol. 106, iss. 2, pp. 194 to 198) with BamHI was ligated downstream of the Prrn-T7G10 sequence. Furthermore, the resulting sequence was linked to a TpsbA-PpsbA-GFP-Trps16 sequence obtained by digesting pLD200-GFP with BamHI and SalI, so that the TpsbA-PpsbA-GFP-Trps16 sequence was arranged downstream of the aadA sequence.

The Prrn-T7G10-aadA-TpsbA-PpsbA-GFP-Trps16 cassette constructed as described above was inserted in the NotI-SalI site of the pNtAP1. Thus, pNtag was obtained (see B in Fig. 2). Moreover, the pPrrn-Bs-TrbcL fragment was inserted in the NotI/BsiWI site of the pNtag. Thus, a tobacco chloroplast transformation vector pNtagBs was obtained (see C in Fig. 2).

### (Example 5)

### <Introduction of Barstar Gene into Chloroplast DNA of Barnase Gene-Introduced Tobacco (Chloroplast Transformation)>

Leaf segments were prepared from each of the barnase gene-introduced line #7 and wildtype tobacco. Then, the obtained leaf segments were placed on a central portion of a MSBN medium with the abaxial side of the leaf segments facing up in such a manner as to draw a circle having a diameter of 3.5 cm. Subsequently, after culturing for 16 hours in the light and at 25°C (unless otherwise specifically indicated regarding the culture condition, the culturing was performed under this condition) for 1 day, the barstar gene-introduction vector pNtagBs or pNtag was introduced by a particle gun method.

Note that, for the particle gun, 0.6-µm gold particles and a 1,100 psi rupture disc were used, and the distance from the rupture disc to samples were set to 6 cm.

After the genes were introduced in this manner, the tobacco leaf segments were turned over on the MSBN medium, and cultured in the dark at 25°C for 2 days. Then, the leaf segments were transplanted to a MSBN medium supplemented with 500 mgL⁻¹ of spectinomycin (MSBN-SPC medium), and cultured for selection. Note that the medium was replaced every 2 weeks. After 4 to 6 weeks, adventitious shoots differentiated from the leaf segments were transplanted to a MS medium supplemented with 500 mgL⁻¹ of spectinomycin, and individuals observed to have root developments were obtained as spectinomycin-resistant individuals.

Subsequently, the spectinomycin-resistant individuals were observed using a fluorescence microscope. Note that, for the GFP fluorescence observation, a GFP2 filter was used (480-nm excitation filter /510-nm barrier filter; Leica MZ 16FA, manufactured by Leica Microsystems GmbH).

As a result, GFP fluorescence was observed only in chloroplasts in the leaf cells. This revealed that the genes had been introduced into the chloroplast DNA, and the GFP gene was expressed. Moreover, although individuals believed to be homoplasmic with almost all the chloroplasts showing the GFP fluorescence were also obtained, many individuals were heteroplasmic individuals (individuals composed of heteroplasmic plant cells or chimeric individuals), in which non-transformed wildtype chloroplasts were also observed.

Next, PCR and Southern blotting analyses were performed using a DNA extracted from the tobacco having GFP fluorescence in the chloroplasts. The results revealed that these individuals were chloroplast transformants having the genes inserted in the target sites of the chloroplast DNA. Meanwhile, it was also revealed that both the transformed recombinant chloroplast DNA and the wildtype chloroplast DNA were present in the heteroplasmic individuals.

Hereinafter, tobacco having the barnase gene introduced in the nucleus and the pNtag introduced in the chloroplast may also be referred to as "barnase/GFP". Tobacco having the barnase gene introduced in the nucleus and the pNtagBs introduced in the chloroplast may also be referred to as "barnase/barstar". Moreover, tobacco having the wildtype nucleus and the pNtagBs introduced in the chloroplast may also be referred to as "WT/barstar".

### (Example 6)

### <Inhibition of Barnase from Disrupting Chloroplasts by Barstar Expression>

Whether or not barstar was capable of inhibiting barnase from disrupting chloroplasts was examined using the homoplasmic barnase/barstar. Specifically, leaf segments of 5 mm square were prepared from the barnase/barstar and the barnase/GFP, and six segments from each were cultured on a MSBN-SPC medium supplemented with 0 µM or 5 µM estradiol. Then, the medium was replaced once after 2 weeks, and observed after 4 weeks. Fig. 4 shows the obtained result.

As apparent from the result shown in Fig. 4, in a case barnase was induced by estradiol also, the re-differentiated adventitious shoot formation was observed in the barnase/barstar. On the other hand, in the barnase/GFP used as the control, the re-differentiated adventitious shoot formation was suppressed by the inducible expression of barnase. In addition, although unillustrated, in the WT/barstar, the re-differentiated adventitious shoots were formed regardless of estradiol. This suggests that the expression of barstar in chloroplasts has no influence on the formations of plants and re-differentiated adventitious shoots. Thus, it was verified that the action of barnase was inhibited by barstar in the chloroplasts of the barnase/barstar.

Next, protoplasts were prepared from each leaf of the re-differentiated adventitious shoots formed by the 5-µM estradiol treatment, and chloroplasts in the cells were observed. Fig. 5 shows the obtained result.

As apparent from the result shown in Fig. 5, the disruption caused by inhibiting the chloroplast function was observed in the barnase/GFP, but the chloroplast disruption by barnase was suppressed in the barnase/barstar.

The above results revealed that the inhibition of the chloroplast function by barnase was successfully suppressed by expressing barstar in the chloroplasts. It was further revealed that the interaction between barnase and barstar functioned also in chloroplasts that had not been utilized in literatures so far.

### (Example 7)

### <Promotion of Homoplasmy by Barnase/Barstar System>

In order to examine whether or not a proportion of homoplasmic secondary adventitious shoots re-differentiated from heteroplasmic leaves was increased by using the barnase/barstar system, the following experiment was conducted.

Experimental materials used were four independent lines from each of the barnase/barstar and the control WT/barstar, having heteroplasmic leaves, in which both the wildtype chloroplast having no GFP fluorescence and the transformed chloroplast were present. Four leaf segments of 5 mm square were prepared from two heteroplasmic leaves of each line. Four segments equally from the eight segments in total were cultured on a MSBN-SPC medium supplemented with 0 µM or 10 µM estradiol. The medium was replaced every 2 weeks. Then, all the secondary adventitious shoots that re-differentiated at 4 weeks thereafter were independently cut, and GFP fluorescence in the leaves of each of the adventitious shoots was observed. Fig. 6 shows the obtained result. Moreover, only adventitious shoots having GFP fluorescence observed in all the chloroplasts in the entire surface of the adventitious shoots were regarded as homoplasmic adventitious shoots. A proportion of the homoplasmic adventitious shoots produced was examined. Fig. 7 shows the obtained result.

As apparent from the results shown in Figs. 6 and 7, when the four barnase/barstar lines were cultured on the medium supplemented with estradiol, the proportion of the homoplasmic adventitious shoots was 34.8%, which was approximately 3 times as high as that (13.7%) in the medium supplemented with no estradiol. Meanwhile, as to the WT/barstar having no barnase gene, the proportion of the homoplasmic adventitious shoots without the estradiol treatment was 6.2%, while that with estradiol was 2.9%.

As described above, it was revealed that when the estradiol treatment was performed, the barnase/barstar had the proportion of the homoplasmic adventitious shoots produced 5 to 10 times as high as that of the WT/barstar. It was demonstrated that barnase positively destroyed the wildtype chloroplasts, and that the use of the barnase/barstar system having barstar-expressing transformed chloroplasts remained made it possible to increase the proportion of the homoplasmic adventitious shoots during the adventitious shoot re-differentiation.

### (Example 8)

### <Promotion of Homoplasmy by Barnase/Barstar Syste m>

Leaf segments of primary re-differentiated individuals Bn/Bs-6 of the barnase/barstar showing heteroplasmy and leaf segments of primary re-differentiated individuals WT/Bs-5 of the WT/barstar showing heteroplasmy were cultured on MSBN-SPC media supplemented with 10 µM estradiol for 4 weeks. Note that the notation such as "Bn/Bs-6" and "WT/Bs-5" indicates "nucleus type/chloroplast type-individual identification number." Then, all of resulting secondary adventitious shoots were independently cut and arranged on a MS-SPC medium to observe GFP fluorescence. Fig. 8 shows the obtained result.

As apparent from the result shown in Fig. 8, many recombinant chloroplasts showing GFP fluorescence were present in the secondary adventitious shoots obtained from the Bn/Bs-6, and strong GFP fluorescence was observed in the entire surface of each secondary adventitious shoot (see A in Fig. 8). On the other hand, wildtype chloroplasts were also present in the secondary adventitious shoots obtained from the WT/Bs-5, and weak GFP fluorescence was observed in the entire surface of the adventitious shoots (see B in Fig. 8).

From the above also, it was demonstrated that the use of the barnase/barstar system made it possible to promote the homoplasmy of cells composing each adventitious shoot during the adventitious shoot re-differentiation, and to increase the proportion of adventitious shoots composed only of homoplasmic cells.

### (Example 9)

### <Detection of Recombinant Chloroplast DNA in Secondary Re-Differentiated Individuals Obtained from Barnase/Barstar System>

In order to confirm that the proportion of cells, which became homoplasmic by using the barnase/barstar system, is high in a plant, that is, to confirm that the plant had the recombinant chloroplast DNA in a high proportion, the genotype of the chloroplast DNA of the secondary re-differentiated individuals was examined by a Southern blotting analysis.

Specifically, the secondary adventitious shoots produced in Example 8 (see Fig. 8) were transplanted to rooting media supplemented with spectinomycin (500 mgL⁻¹) and cultured for 3 weeks, and ones having root developments were obtained as secondary re-differentiated individuals. Then, the Southern blotting analysis was performed on these secondary re-differentiated individuals according to an ordinary method under conditions as follows.

First, total DNA was extracted from an analysis material, 1.5 µg of the DNA was digested using BglII, and the detection was performed using an endogenous chloroplast DNA-derived sequence NttrnIout probe. Note that when the total DNA was digested with BglII and the NttrnIout probe was used, it was expected that a 4.5-kb signal and a 7.8-kb signal were detected from the wildtype chloroplast and the recombinant chloroplast, respectively (see Fig. 9).

Moreover, all the secondary re-differentiated individuals obtained from the primary re-differentiated individuals Bn/Bs-6, which were of the #7 line having the pNtagBs introduced in the chloroplast and the barnase gene inserted in the nucleus, were used as the analysis material. Further, the analysis was performed also on all the secondary re-differentiated individuals obtained from the primary re-differentiated individualsWT/Bs-5, which were tobacco having the wildtype nucleus and chromosome and the pNtagBs introduced in the chloroplast. Furthermore, chloroplast transformation materials wild type individuals (WT/WT) and #7 line (barnase/WT), and the primary re-differentiated individuals Bn/Bs-6 and WT/Bs-5 were used as controls. Figs. 10 to 13 show the obtained results.

As apparent from the result shown in Figs. 10 and 11, only the 4.5-kb signal was detected in the wildtype (WT/WT) and the #7 line (barnase/WT). This indicated that these plants had only the wildtype chloroplast. Meanwhile, in the primary re-differentiated individuals Bn/Bs-6 and WT/Bs-5, the wildtype 4.5-kb signal and the recombinant 7.8-kb signal were detected at equivalent levels. This indicated the heteroplasmy.

The estradiol treatment was performed on secondary adventitious shoots prepared from such heteroplasmic primary re-differentiated individuals Bn/Bs-6, and 23 secondary re-differentiated individuals obtained by using the barnase/barstar system were subjected to the analysis. As a result, as apparent from the result shown in Fig. 12, the recombinant signal was detected in all the 23 individuals. In 9 individuals among these, the expected recombinant 7.8-kb signal was strongly detected, indicating that the plants were composed of homoplasmic cells in quite a high proportion.

On the other hand, as apparent from the result shown in Fig. 13, in 31 secondary re-differentiated individuals obtained from the primary re-differentiated individuals WT/Bs-5 not having the barnase gene in the nucleus, the recombinant DNA signal was strongly detect only in one individual (WT/Bs-5-28), but the band size was different from the expected position. Among the other 30 individuals, some had the recombinant signal detected equivalently to the wildtype, and others had the wildtype signal detected strongly. Note that although some individuals had the recombinant signal not clearly detected, GFP fluorescence was observed in the chloroplasts when leaves of these were observed. This suggested that these individuals had the recombinant DNA at the detection limit of the Southern analysis or below.

The above results revealed that when secondary re-differentiated individuals were produced from leaf segments of primary re-differentiated individuals showing heteroplasmy, the use of the barnase/barstar system increased the proportion of homoplasmic cells in the plants, and improved the efficiency of producing secondary re-differentiated individuals with the heteroplasmy eliminated.

### (Example 10)

### <Slight Promotion of Homoplasmy Without Barnase Gene Induction Treatment>

As described above, the barnase/barstar system efficiently promoted the homoplasmy. On the other hand, even if the expression of the barnase gene was induced, a slight (two-fold) promotion of the homoplasmy was observed in the barnase/barstar in comparison with the WT/barstar (see Fig. 7). Although the barnase gene was linked to the estradiol-inducible promoter in the barnase gene-introduced tobacco, it was observed by an RT-PCR analysis that the barnase gene was slightly expressed non-inducibly without the estradiol treatment (see Fig. 14). Thus, even without the estradiol treatment, the barnase/barstar system slightly functioned and the promotion of the homoplasmy was observed presumably due to the influence of the non-inducible barnase expression.

### [Industrial Applicability]

As has been described above, the present invention enables efficient production of highly homoplasmic plant cells, in which most of plastids and the like are transformed, highly homoplasmic plants composed mostly of the plant cells, and so forth. Particularly, in transforming plastids, a total level of expression of introduced exogenous genes per cell of an equivalent volume is increased. This makes it possible to produce plastid-transformed plants having a high substance-producing ability.

Thus, the present invention can be effectively utilized for preventing gene flow by methods for transforming plastids and the like, and in substance productionsystemsforbiomass,functionalfood materials, and pharmaceutical materials.

### [Sequence Listing Free Text]

SEQ ID NOs: 1 to 12, and 15 to 18
<223> Artificially synthesized primer sequence SEQ ID NO: 13
<223> Artificially synthesized oligonucleotide sequence

## Claims

1. A method for transforming organelles having an own genomic DNA in a plant, the method comprising the steps of:
expressing in plant cells a fusion protein containing a function inhibiting factor of the organelles and a transit signal peptide to the organelles;
introducing into the genomic DNA of the organelles of the plant cells an expression cassette comprising a DNA encoding a restoring factor of the organelles and a DNA encoding a factor desired to be expressed in the organelles; and
allowing the function inhibiting factor to destroy the organelles, in which the expression cassette is not introduced, in the plant cells.

2. The method according to claim 1, wherein the function inhibiting factor is barnase, and the restoring factor is barstar.

3. The method according to any one of claims 1 and 2, wherein the organelles are plastids.

4. The method according to any one of claims 1 to 3, wherein the expression of the function inhibiting factor is induced in response to a stimulus.

5. At least one vector selected from the following (a) and (b) used in the method according to any one of claims 1 to 4:
(a) a vector comprising
a promoter capable of functioning in the plant cells, and
a DNA operably linked to the promoter and encoding the fusion protein; and
(b) a vector comprising
an expression cassette comprising a first promoter capable of functioning in the organelles, the DNA operably linked to the first promoter and encoding the restoring factor, a second promoter capable of functioning in the organelles, and the DNA operably linked to the second promoter and encoding the factor desired to be expressed in the organelles, and
DNA sequences located on both sides of the expression cassette and having a homology with the genomic DNA specific to the organelles.

6. A transformant produced by the method according to any one of claims 1 to 4.

7. A transformant comprising at least one DNA selected from the following (a) and (b):
(a) a promoter capable of functioning in the plant cells and a DNA operably linked to the promoter and encoding the fusion protein; and
(b) a first promoter capable of functioning in organelles having an own genomic DNA in a plant, a DNA operably linked to the first promoter and encoding the restoring factor, a second promoter capable of functioning in the organelles, and a DNA operably linked to the second promoter and encoding the factor desired to be expressed in the organelles.

8. A transformant that is any one of a progeny and a clone of the transformant according to any one of claims 6 and 7.

9. The transformant according to any one of claims 6 to 8, wherein the transformant is any one selected from the group consisting of a plant cell, a plant tissue, a plant organ, a plant, and a seed.

10. A processed product of the transformant according to any one of claims 6 to 9, the processed product comprising an expression product of the factor desired to be expressed in the organelles.
